(19) 

**Europäisches Patentamt**
**European Patent Office**
**Office européen des brevets**

(11) **EP 4 471 414 A1**

(12) **EUROPEAN PATENT APPLICATION**
published in accordance with Art. 153(4) EPC

(43) Date of publication:
04.12.2024 Bulletin 2024/49

(21) Application number: 23747056.2

(22) Date of filing: 26.01.2023

(51) International Patent Classification (IPC):
*G01N 25/18* (2006.01)

(52) Cooperative Patent Classification (CPC):
G01N 25/18

(86) International application number:
PCT/JP2023/002541

(87) International publication number:
WO 2023/145846 (03.08.2023 Gazette 2023/31)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 27.01.2022 JP 2022011351

(71) Applicant: **National University Corporation Tokai National
Higher Education and Research System
Nagoya-shi, Aichi 464-8601 (JP)**

(72) Inventors:
• **NAGANO Hosei**
**Nagoya-shi Aichi 464-8601 (JP)**
• **AL ASLI Abdulkareem**
**Nagoya-shi Aichi 464-8601 (JP)**

(74) Representative: **Kuhnen & Wacker
Patent- und Rechtsanwaltsbüro PartG mbB
Prinz-Ludwig-Straße 40A
85354 Freising (DE)**

(54) **DEVICE, METHOD, AND PROGRAM**

(57) A device of the present invention includes: an irradiation unit configured to periodically irradiate an object with light to heat the object; an output power switching unit configured to switch an output power of light with which the object is irradiated by the irradiation unit at least between first and second output powers respectively providing first and second maximum output powers during one period; a first temperature distribution acquisition unit configured to obtain a first temperature distribution of the object that is a temperature distribution of the object heated by light of the first output power; a second temperature distribution acquisition unit configured to obtain a second temperature distribution of the object that is a temperature distribution of the object heated by light of the second output power; and an identification unit configured to identify information about thermal conductivity of the object based on the first and second temperature distributions.

Fig.1

**Description**

Technical Field

The present invention relates to a device, a method, and a program.

Background Art

Patent Document 1 discloses a thermal conductivity measurement device including a refrigerant storage tank surrounding a test specimen. The device is configured to cool the test specimen with refrigerant introduced into the refrigerant storage tank to measure thermal conductivity of the test specimen at a temperature at or below room temperature. The device accurately measures the thermal conductivity of the test specimen by filling the refrigerant storage tank with a porous body in which the refrigerant can be dispersed and by homogenizing the temperature of the test specimen in the refrigerant storage tank by means of the refrigerant dispersed in the porous body.

Citation List

Patent Literature

Patent Document 1: Japanese Patent Application Laid-Open Publication No. 2002-90322

Summary of Invention

Technical Problem

A need has existed for the ability to identify information about thermal conductivity of a measurement object in a non-contact manner in order to, for example, determine the quality of products in manufacturing processes.
The techniques disclosed herein aim to identify information about thermal conductivity of a measurement object using a novel approach.

Solution to Problem

With the above object in view, the techniques disclosed herein relate to a device including: an irradiation unit configured to periodically irradiate an object with light to heat the object; an output power switching unit configured to switch an output power of the light with which the object is irradiated by the irradiation unit at least between a first output power and a second output power, the first output power providing a first maximum output power during one period, the second output power providing a second maximum output power during one period; a first temperature distribution acquisition unit configured to obtain a first temperature distribution of the object, the first temperature distribution being a temperature distribution of the object heated by the light of the first output power; a second temperature distribution acquisition unit configured to obtain a second temperature distribution of the object, the second temperature distribution being a temperature distribution of the object heated by the light of the second output power; and an identification unit configured to identify information about thermal conductivity of the object based on the first temperature distribution and the second temperature distribution.
The identification unit may be configured to: obtain a first temperature amplitude distribution of the object based on the first temperature distribution, the first temperature amplitude distribution being a temperature amplitude distribution of the object heated by the light of the first output power; obtain a second temperature amplitude distribution of the object based on the second temperature distribution, the second temperature amplitude distribution being a temperature amplitude distribution of the object heated by the light of the second output power; and identify the information about thermal conductivity of the object based on the first temperature amplitude distribution and the second temperature amplitude distribution.
The identification unit may be configured to: obtain a first maximum temperature distribution of the object based on the first temperature distribution, the first maximum temperature distribution being a distribution of maximum temperatures at respective locations on the object heated by the light of the first output power; obtain a second maximum temperature distribution of the object based on the second temperature distribution, the second maximum temperature distribution being a distribution of maximum temperatures at respective locations on the object heated by the light of the second output power; and identify the information about thermal conductivity of the object based on the first maximum temperature distribution and the second maximum temperature distribution.
The device may further include a thermal diffusivity identification unit configured to identify information about thermal diffusivity of the object based on responses of changes over time in the temperature distribution of the object heated by the light of the first output power.

The device may further include a specific heat identification unit configured to identify information about specific heat of the object based on the information about thermal conductivity of the object identified by the identification unit.

The device may further include a fatigue information identification unit configured to identify information about fatigue of the object based on the information about thermal conductivity of the object identified by the identification unit.

The irradiation unit may be configured to irradiate the object over a wider range than a region of the object in which the temperature distribution is measured, and the identification unit may be configured to identify information about through-plane thermal conductivity of the object.

The irradiation unit may be configured to irradiate a region of the object extending in one direction on a surface of the object, and the identification unit may be configured to identify information about in-plane thermal conductivity of the object.

The irradiation unit may be configured to switch between a first mode and a second mode, the first mode being a mode in which the irradiation unit irradiates the object over a wider range than a region of the object in which the temperature distribution is measured, the second mode being a mode in which the irradiation unit irradiates a region of the object extending in one direction on a surface of the object, and the identification unit may be configured to identify information about through-plane thermal conductivity of the object in the first mode and to identify information about in-plane thermal conductivity of the object in the second mode.

From another standpoint, the techniques disclosed herein relate to a method including: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and identifying information about thermal conductivity of the object based on the first temperature distribution and the second temperature distribution.

From still another standpoint, the techniques disclosed herein relate to a program for causing a computer to execute functions of: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and identifying information about thermal conductivity of the object based on the first temperature distribution and the second temperature distribution.

From still another standpoint, the techniques disclosed herein relate to a device including: an irradiation unit configured to periodically irradiate an object with light to heat the object; an output power switching unit configured to switch an output power of the light with which the object is irradiated by the irradiation unit at least between a first output power and a second output power, the first output power providing a first maximum output power during one period, the second output power providing a second maximum output power during one period; a first temperature distribution acquisition unit configured to obtain a first temperature distribution of the object, the first temperature distribution being a temperature distribution of the object heated by the light of the first output power; a second temperature distribution acquisition unit configured to obtain a second temperature distribution of the object, the second temperature distribution being a temperature distribution of the object heated by the light of the second output power; and an identification unit configured to identify information about specific heat of the object based on the first temperature distribution and the second temperature distribution.

From still another standpoint, the techniques disclosed herein relate to a method including: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and identifying information about specific heat of the object based on the first temperature distribution and the second temperature distribution.

From still another standpoint, the techniques disclosed herein relate to a program for causing a computer to execute functions of: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and identifying information about specific heat of the object based on the first temperature distribution and the second temperature distribution.

From still another standpoint, the techniques disclosed herein relate to a device including: an irradiation unit configured to periodically irradiate an object with light to heat the object; an output power switching unit configured to switch an output power of the light with which the object is irradiated by the irradiation unit at least between a first output power and a second output power, the first output power providing a first maximum output power during one period, the second output power providing a second maximum output power during one period; a first temperature distribution acquisition unit configured to obtain a first temperature distribution of the object, the first temperature distribution being a temperature distribution of the object heated by the light of the first output power; a second temperature distribution acquisition unit configured to obtain a second temperature distribution of the object, the second temperature distribution being a temperature distribution of the object heated by the light of the second output power; and an identification unit configured to, based on the first temperature distribution and the second temperature distribution, calculate thermal conductivity of the object to identify information about fatigue of the object.

From still another standpoint, the techniques disclosed herein relate to a method including: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and based on the first temperature distribution and the second temperature distribution, calculating thermal conductivity of the object to identify information about fatigue of the object.

From still another standpoint, the techniques disclosed herein relate to a program for causing a computer to execute functions of: periodically irradiating an object with light to heat the object; switching an output power of the light with which the object is irradiated at least between a first output power and a second output power, the first output power providing a first maximum output power during one period, the second output power providing a second maximum output power during one period; obtaining a first temperature distribution of the object, the first temperature distribution being a temperature distribution of the object heated by the light of the first output power; obtaining a second temperature distribution of the object, the second temperature distribution being a temperature distribution of the object heated by the light of the second output power; and based on the first temperature distribution and the second temperature distribution, calculating thermal conductivity of the object to identify information about fatigue of the object.

Advantageous Effects of Invention

The techniques disclosed herein can identify information about thermal conductivity of a measurement object using a novel approach.

Brief Description of Drawings

FIG. 1 illustrates a schematic configuration of a thermophysical property evaluation device according to a first embodiment.
FIG. 2 illustrates a functional configuration of a computer.
FIG. 3 illustrates an example hardware configuration of the computer.
FIGS. 4(A) and 4(B) illustrate the principles of measuring thermophysical properties in the first embodiment.
FIGS. 5(A) and 5(B) illustrate a model of the measurement principles in the first embodiment.
FIG. 6 illustrates the principle of measuring a specific heat capacity in the first embodiment.
FIG.7 is a flowchart of a fatigue evaluation and determination operation by the thermophysical property evaluation device.
FIG.8 is a flowchart of a fatigue evaluation process by the thermophysical property evaluation device.
FIGS. 9(A) through 9(E) illustrate first measurement results.
FIGS. 10(A) through 10(D) illustrate second measurement results.

FIGS. 11(A) through 11(E) illustrate the second measurement results.

FIG. 12 illustrates a schematic configuration of a thermophysical property evaluation device according to a second embodiment.

FIGS. 13(A) and 13(B) illustrate the principles of measuring thermophysical properties in the second embodiment.

FIGS. 14(A) and 14(B) illustrate a model of the measurement principles in the second embodiment.

FIG. 15 illustrates the principle of measuring a specific heat capacity in the second embodiment.

FIGS. 16(A) through 16(C) illustrate third measurement results.

FIG. 17 illustrates a schematic configuration of a thermophysical property evaluation device according to a third embodiment.

FIGS. 18(A) through 18(D) illustrate the principles of measuring thermophysical properties in the third embodiment.

FIG. 19 illustrates the principle of measuring a specific heat capacity in the third embodiment.

FIG. 20 illustrates a schematic configuration of a thermophysical property evaluation device according to a fourth embodiment.

FIGS. 21(A) through 21(D) illustrate the principles of measuring thermophysical properties in the fourth embodiment.

FIG. 22 illustrates the principle of measuring a specific heat capacity.

FIG. 23 illustrates a measurement condition control unit according to variation 1.

Description of Embodiments

Exemplary embodiments are described below with reference to the appended drawings.

<First Embodiment>

<Configuration of Thermophysical Property Evaluation Device 100>

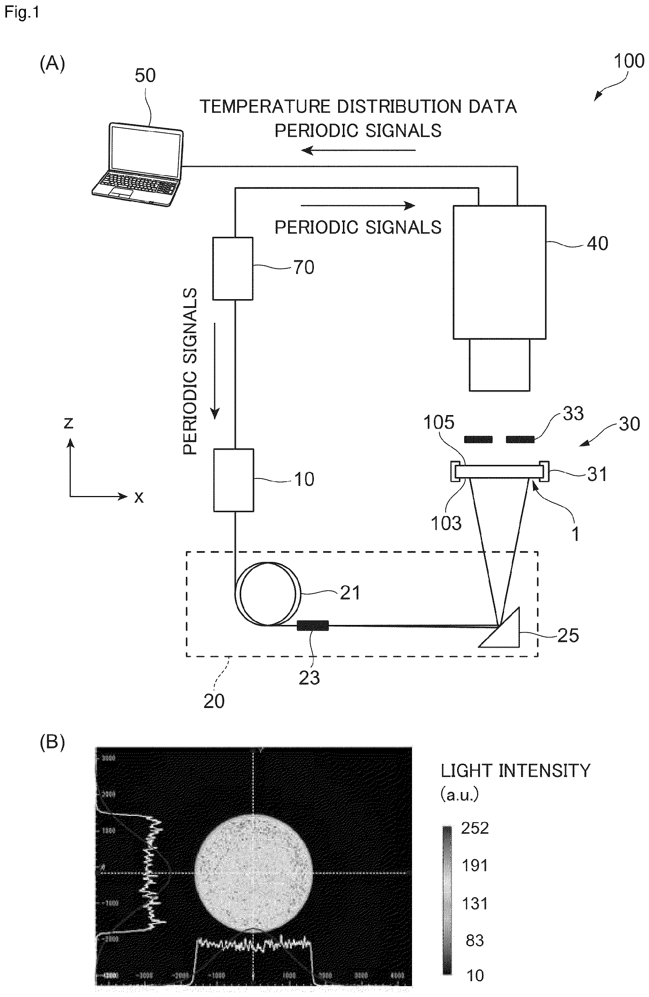

FIG. 1 illustrates a schematic configuration of a thermophysical property evaluation device 100 according to a first embodiment.

Referring to FIG. 1, a configuration of the thermophysical property evaluation device 100 according to the present embodiment is first described below.

As shown in FIG. 1, the thermophysical property evaluation device 100 according to the present embodiment includes a diode laser 10 serving as a light source for heating a sample 1 to be measured (hereinafter the "measurement sample 1"), a light guide 20 for directing a laser beam of the diode laser 10 to the measurement sample 1, a support 30 for supporting the measurement sample 1, an infrared thermographic camera (lock-in thermographic camera) 40 located opposite the measurement sample 1, a computer 50 for receiving signals from the infrared thermographic camera 40, and a periodic signal generator 70 for generating periodic signals and outputting them to the diode laser 10 and the computer 50.

The diode laser 10 and the light guide 20 irradiate a surface of the planar measurement sample 1 with light having a uniform intensity distribution. The diode laser 10 is an area heating light source. The diode laser 10 outputs a so-called multimode diode laser beam (e.g., TEM01) whose transverse mode is not a single mode (TEM00).

The light guide 20 includes a fiber 21 as a transmission path for the laser beam emitted from the diode laser 10, a condenser 23 located at the distal end of the fiber 21 to control the intensity distribution of the laser beam emitted from the fiber 21, and a mirror 25 for reflecting the laser beam emitted from the condenser 23.

The fiber 21 is composed of a multimode fiber capable of propagating laser beams of different spatial modes in a mixed manner. Inside this fiber 21, the incident angle of the laser beam emitted from the diode laser 10 is controlled to split the laser beam into meridional and skew rays. The laser beam emitted from the diode laser 10 repeatedly undergoes multiple reflections inside the fiber 21, resulting in a uniform intensity distribution on the irradiation plane. The fiber 21 in the illustrated example has a core diameter of 100 μm and a length of 3 m.

The condenser 23 is composed of a plurality of lenses or the like and serves as a variable focus condenser optic. The condenser 23 focuses (or diffuses) the laser beam emitted from the fiber 21. The laser beam emitted from the condenser 23 propagates through the space while spreading. Thus, the condenser 23 can be viewed as a beam expander that expands the beam diameter of the laser beam emitted from the fiber 21.

The mirror 25 is composed of a well-known optical mirror, such as a glass substrate coated with a thin metal or dielectric film. The mirror 25 reflects the laser beam emitted from the condenser 23 toward the measurement sample 1.

In the thermophysical property evaluation device 100 configured as above, the laser beam emitted from the diode laser 10 is incident on a first surface 103 of the measurement sample 1 through the fiber 21, the condenser 23, and the mirror 25. The measurement sample 1 is periodically heated by the laser beam. As shown in FIG. 1(B), the laser beam going through the fiber 21, the condenser 23, and the mirror 25 is controlled such that the intensity distribution on the irradiation plane is homogenized into a so-called top-hat type intensity distribution.

The temperature of the measurement sample 1, which is periodically heated by the laser beam from the diode laser 10, is measured by the infrared thermographic camera 40 at a second surface 105 of the measurement sample 1. The infrared thermographic camera 40 receives periodic signals input from the periodic signal generator 70. The temperature distribution data, which is the data of the temperatures measured by the infrared thermographic camera 40, is output to the computer 50. The infrared thermographic camera 40 captures (measures) a predetermined area in the region periodically heated by the diode laser 10 as an infrared image. In other words, the diode laser 10 irradiates, with the laser beam, the entire portion of the measurement sample 1 corresponding to the region observed by the infrared thermographic camera 40. That is, the region of the measurement sample 1 illuminated by the diode laser 10 is larger (wider) than the region observed by the infrared thermographic camera 40.

The support 30 includes a support body 31 for holding ends of the measurement sample 1 and a diaphragm 33 for demarcating a region of the measurement sample 1 to be observed by the infrared thermographic camera 40. The support body 31 is driven by a tensile tester (not shown) to apply a tensile load to the measurement sample 1. The diaphragm 33 defines a region of the measurement sample 1 to be subjected to measurement by the infrared thermographic camera 40. In the illustrated example, a partial region (center region) on the second surface 105 of the measurement sample 1 is subjected to measurement by the infrared thermographic camera 40. This defining of the measurement region of the measurement sample 1 by the diaphragm 33 can improve the accuracy of measurement of the measurement sample 1.

The computer 50, in combination with the infrared thermographic camera 40, continuously captures infrared images and performs computations at predetermined frame rate intervals to create an averaged image based on the amount of temperature change over time (lock-in method). To further illustrate, the data obtained from the infrared thermographic camera 40 is processed by the computer 50 to calculate a through-plane thermal conductivity of the measurement sample 1. The data obtained from the infrared thermographic camera 40 is also processed by the computer 50 to calculate information about thermophysical properties of the measurement sample 1 (details to follow).

Note that the direction along the surface of the measurement sample 1 in FIG. 1, namely the horizontal direction in the figure may be referred to hereinafter as the x-direction. The vertical direction in FIG. 1 may be referred to hereinafter as the z-direction. The direction going into the page of FIG. 1 may be referred to hereinafter as the y-direction.

<Functional Configuration of Computer 50>

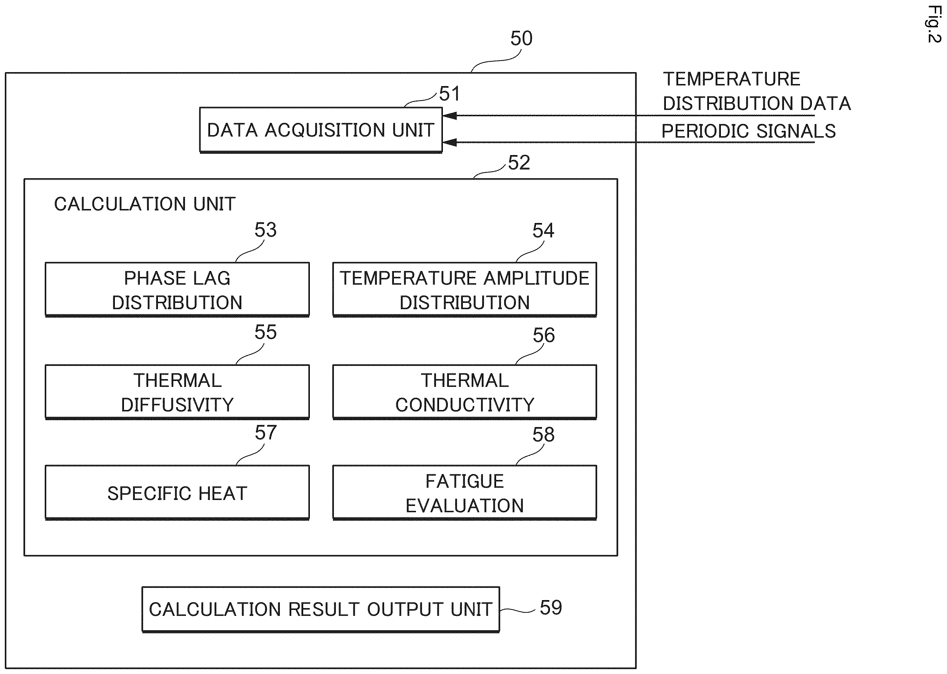

FIG. 2 illustrates a functional configuration of the computer 50.

Referring now to FIGS. 1 and 2, a functional configuration of the computer 50 according to the present embodiment is described.

As shown in FIG. 2, the computer 50 according to the present embodiment includes: a data acquisition unit 51 to obtain temperature distribution data and periodic signals input from the infrared thermographic camera 40 (see FIG. 1); a calculation unit 52 to calculate each measurement based on the temperature distribution data and the periodic signals obtained by the data acquisition unit 51; and a calculation result output unit 59 to output calculation results to a liquid crystal display (not shown) or the like.

The calculation unit 52 includes a phase lag distribution calculation unit 53, a temperature amplitude distribution calculation unit 54, a thermal diffusivity distribution calculation unit 55, a thermal conductivity calculation unit 56, a specific heat calculation unit 57, and a fatigue evaluation calculation unit 58.

The phase lag distribution calculation unit 53 calculates a phase lag distribution based on the temperature distribution data and the periodic signals obtained by the data acquisition unit 51. The temperature amplitude distribution calculation unit 54 calculates a temperature amplitude distribution based on the temperature distribution data and the periodic signals obtained by the data acquisition unit 51. The thermal diffusivity distribution calculation unit 55 calculates a thermal diffusivity distribution based on the phase lag calculated by the phase lag distribution calculation unit 53. The thermal conductivity calculation unit 56 calculates a thermal conductivity based on the temperature amplitude distribution calculated by the temperature amplitude distribution calculation unit 54. The specific heat calculation unit 57 calculates specific heat based on the thermal diffusivity calculated by the thermal diffusivity distribution calculation unit 55 and the thermal conductivity calculated by the thermal conductivity calculation unit 56. The fatigue evaluation calculation unit 58 calculates a fatigue evaluation (described below), which is an evaluation of fatigue of the measurement sample 1 (see FIG. 1), based on the specific heat calculated by the specific heat calculation unit 57.

The thermal diffusivity distribution calculation unit 55 calculates the thermal diffusivity based on equation (2) described below. The thermal conductivity calculation unit 56 calculates the thermal conductivity based on equation (6) described below. The specific heat calculation unit 57 calculates the specific heat based on equation (1) described below.

The computer 50 of the present embodiment evaluates the measurement sample 1 (see FIG. 1), i.e., evaluates the thermophysical properties and fatigue (described below) of the measurement sample, based on the temperature distribution of the measurement sample 1 detected by the infrared thermographic camera 40.

The thermophysical properties as referred to herein include the thermal diffusivity, thermal conductivity, and specific heat. To further illustrate, the computer 50 of the present embodiment calculates at least one of the thermal diffusivity distribution, thermal conductivity distribution, and specific heat distribution of the measurement sample 1 (see FIG. 1) based on the temperature distribution of the measurement sample 1 detected by the infrared thermographic camera 40. The thermophysical properties may also include fatigue (described below).

<Fatigue Evaluation>

The fatigue evaluation in the present embodiment is now described.

First of all, a fatigue condition refers to the degree of material alteration that occurs as a result of the measurement sample 1 being continuously or repeatedly subjected to cyclic stress. Evaluation of the fatigue condition refers to evaluation of the degree of fatigue. As the fatigue progresses, crack initiation due to fatigue damage, fatigue crack propagation, breakage, etc. occur in the measurement sample 1.

Hereinafter, fatigue properties of carbon fiber-reinforced composite materials as an example of the measurement sample 1 are described first, followed by a description of the principles of the measurement method in the present embodiment.

Carbon fiber-reinforced composite materials (CFRPs), which are an example of the measurement sample 1, are expected to find applications in, for example, transportation and aerospace industries by virtue of their advantages such as high specific strength, corrosion resistance, and fatigue resistance properties. Here, characteristics of carbon fiber-reinforced composite materials associated with fatigue, i.e., fatigue properties, vary greatly depending on the manufacturing quality and operating environment. Therefore, it is required to understand the fatigue properties of carbon fiber-reinforced composite materials. Such understanding of the fatigue properties is expected to, for example, extend the designed lifetime of products.

Repeated application of loads to a carbon fiber-reinforced composite material can cause a crack (fatigue crack) in the carbon fiber-reinforced composite material. As the fatigue crack grows in length, it may lead to delamination or fiber rupture inside the carbon fiber-reinforced composite material, eventually leading to fatigue breakage of the entire material. In addition, slight delamination (micro delamination) may occur and grow to cause larger delamination. The origin of such delamination is believed to be stress risers, such as microvoids (tiny voids) inside the material that occur during manufacture, portions where a resin layer is thin due to proximity between fibers, and inter-layer portions. Microcracks and micro delamination are believed to occur and grow from these stress risers, leading to macroscopic fatigue cracks and delamination. By quantifying this process, it may be possible to diagnose the fatigue properties or fatigue condition of the material.

Known methods for detecting the origin of fatigue cracks in carbon fiber-reinforced composite materials and their subsequent growth patterns include image analysis and X-ray CT methods. However, the image analysis method can only extract information about the surface layer of a sample and cannot evaluate the interior of the sample. Meanwhile, X-ray CT, which observes small regions, requires an enormous amount of time to evaluate an entire sample. In addition, X-ray CT requires cutting out samples, so that it is difficult to observe large components using this technique. Hence, in the present embodiment, the fatigue condition of the measurement sample 1 is calculated by measuring at least one of the macro-scale thermal diffusivity distribution, thermal conductivity, and specific heat, using non-contact laser heating.

To further illustrate, microcracks and micro delamination in the measurement sample 1 are accompanied by occurrence of a crack interface inside the measurement sample 1. This interface then acts as a thermal resistance, potentially resulting in local changes in the thermal diffusivity, thermal conductivity, and specific heat of the measurement sample 1. To further illustrate, for example, the presence of microcracks may be detected by measuring the in-plane thermal diffusivity, thermal conductivity, and specific heat of the measurement sample 1. In the present embodiment, these changes in thermal diffusivity, thermal conductivity, and specific heat are utilized to quantify the growth trend of microcracks and micro delamination with the use of laser beam, thereby allowing for diagnosis of the fatigue condition of the measurement sample 1. This may allow for quantification of the fatigue condition of the measurement sample 1 in a nondestructive and more extensive manner.

The present embodiment enables detection of growth trends of microcracks and micro delamination that, for example, are not visually recognizable, making it possible to detect an initial degradation of the measurement sample 1. While the microcracks and micro delamination have been discussed above, material alterations that occur inside the measurement sample 1 are not limited to these. Other material alterations include, for example, lattice defects and bond breaks that occur inside the measurement sample 1. In the present embodiment, changes in thermal diffusivity, thermal conductivity, and specific heat, which are examples of the thermophysical property value, may be utilized to quantify these material alterations as well.

The fatigue evaluation of the measurement sample 1 in the present embodiment is performed by measuring at least one of the thermal diffusivity, thermal conductivity, and specific heat, as described above. For example, the specific heat in an undamaged condition is compared with that after a predetermined number of applications of loads, and the difference in specific heat, or more specifically, the amount of change in specific heat is evaluated as a function of the number of load cycles. The larger this evaluation function, the more fatigue has progressed, i.e., the more pronounced the fatigue is. To further illustrate, for example, a threshold of the evaluation function may be defined in advance, and comparison with this threshold may be used to evaluate whether a predetermined fatigue condition has been reached. In other words, the fatigue condition can be diagnosed by monitoring the evaluation function.

In the present embodiment, the specific heat distribution of the measurement sample 1 can also be measured. And a portion with different specific heat than other portions can be diagnosed as having fatigue in progress. In other words, in the present embodiment, the fatigue condition can be diagnosed by monitoring the specific heat distribution in the measurement sample 1.

<Hardware Configuration of Computer 50>

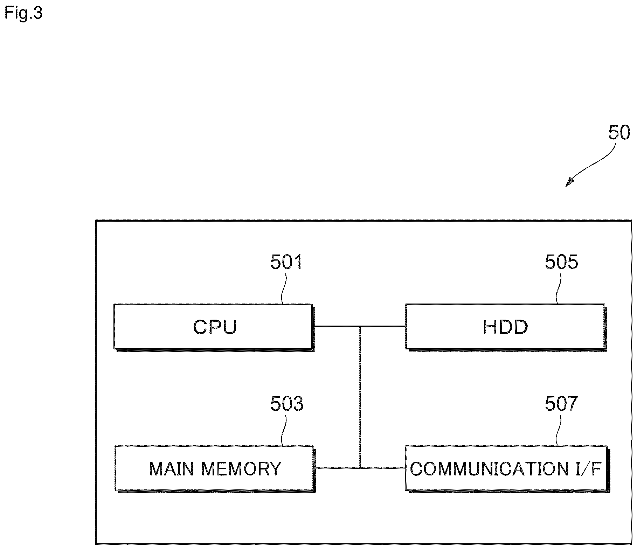

FIG. 3 illustrates an example hardware configuration of the computer 50.

As shown in FIG. 3, the computer 50 includes a central processing unit (CPU) 501 as an arithmetic means, and a main memory 503 and a hard disk drive (HDD) 505 as storage means. The CPU 501 executes various programs such as an operating system (OS) and application software. The main memory 503 is a storage area for storing various programs and data used in the execution of the programs, etc. The HDD 505 is a storage area for storing input data for various programs and output data from various programs, etc. These components of the computer 50 implement the functions described in, among others, FIG. 2 above.

The computer 50 includes a communication interface (communication I/F) 507 for communication with external devices such as the infrared thermographic camera 40. The programs to be executed by the CPU 501 (e.g., the program for calculating the thermophysical properties described above) may be stored in the main memory 503 in advance, may be stored in a storage medium such as a CD-ROM and provided to the CPU 501, or may be provided to the CPU 501 via a network (not shown).

<Measurement Principles>

The principles of measuring thermophysical properties in the first embodiment are now described.

<Specific Heat>

First, the equation to calculate specific heat in the present embodiment is described. The specific heat Cp is a function of thermal conductivity k, thermal diffusivity D, and density $\rho$, as expressed in equation (1) below.
[Equation 1]

$$c_{\mathrm{p}} = k/\rho . D \qquad \cdots (1)$$

In the present embodiment, the specific heat Cp is calculated based on the above equation (1). Specifically, the thermal conductivity k is measured by the thermophysical property evaluation device 100, which employs a lock-in thermography periodic heating method. The thermal diffusivity D is also measured by the thermophysical property evaluation device 100. The density $\rho$ is also measured by any well-known technique, such as the Archimedes method. Thus, the specific heat Cp is calculated based on the above equation (1).

When the density $\rho$ is not measured, the thermal conductivity k and the thermal diffusivity D are measured by the thermophysical property evaluation device 100, and a volumetric specific heat capacity pCp is calculated based on the above equation (1).

While the following discusses measuring the thermal diffusivity D using the lock-in thermographic periodic heating method via the thermophysical property evaluation device 100, the measurement method is not limited to this. Any well-known measurement method capable of measuring the thermal diffusivity D may be employed.

<Through-Plane Specific Heat Distribution>

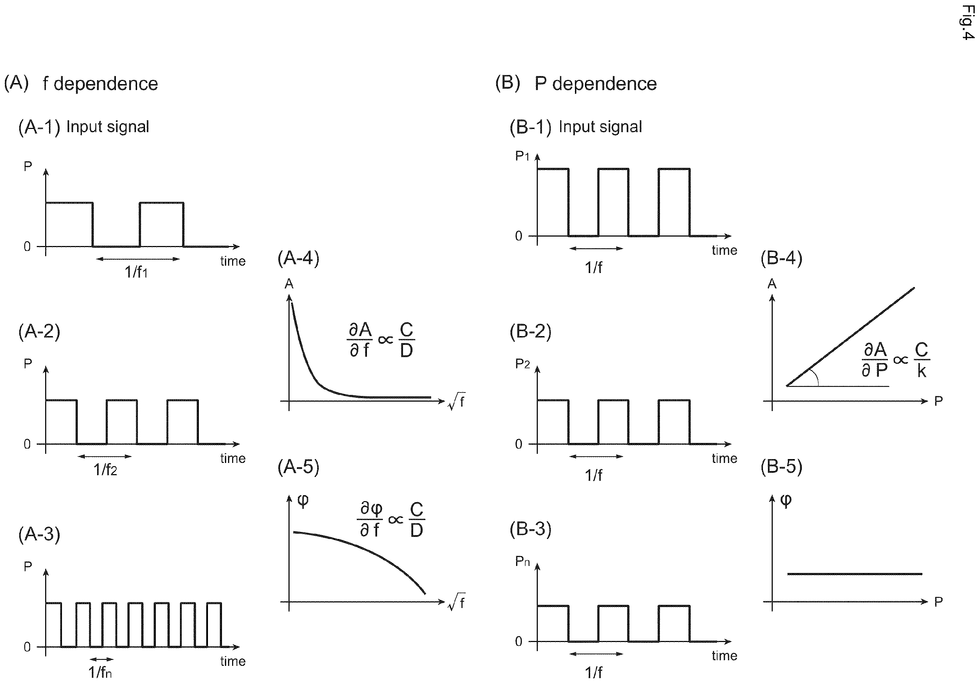

FIGS. 4(A) and 4(B) illustrate the principles of measuring thermophysical properties in the first embodiment, where FIG. 4(A) illustrates the principle of measuring a thermal diffusivity, and FIG. 4(B) illustrates the principle of measuring a thermal conductivity.

The measurement principles in the present embodiment are now outlined with reference to FIGS. 4(A) and 4(B). Here we discuss measuring a specific heat distribution of the measurement sample 1 in the through-plane direction.

First, as shown in FIG. 4(A), the thermal diffusivity is measured using the thermophysical property evaluation device 100. Specifically, a frequency dependence of the temperature amplitude (see FIG. 4(A-4)) and a frequency dependence of the phase lag (see FIG. 4(A-4)) are measured by periodically heating the measurement sample 1 with the laser beam emitted from the diode laser 10 while varying its frequency but keeping its power constant as shown in FIGS. 4 (A-1) through 4(A-3). A thermal diffusivity distribution is then calculated from the measured frequency dependences of the temperature amplitude and the phase lag.

Also, a power dependence of the temperature amplitude (see FIG. 4(B-4)) is measured by periodically heating the measurement sample 1 with the laser beam emitted from the diode laser 10 while varying its power but keeping its frequency constant as shown in FIGS. 4(B-1) through 4(B-3). A thermal conductivity distribution is then calculated from the measured power dependence of the temperature amplitude. In other words, in the present embodiment, the thermal conductivity distribution is determined from the relationship between the temperature amplitude decay and the laser power.

Then, using the measured thermal diffusivity distribution and thermal conductivity distribution as well as a density distribution measured by a well-known technique, a specific heat distribution is calculated from the above equation (1).

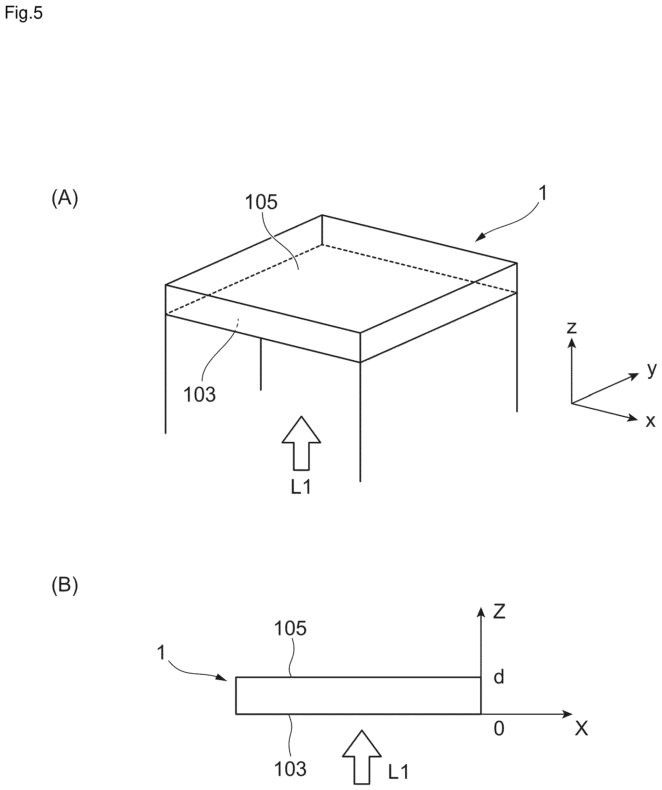

FIGS. 5(A) and 5(B) illustrate a model of the measurement principles in the first embodiment, where the figures depict heating conditions.

The measurement principles in the present embodiment are now detailed with reference to FIGS. 5(A) and 5(B).

As shown in FIGS. 5(A) and 5(B), the generally planar measurement sample 1 has a thickness d. This measurement sample 1 has a thermal conductivity k and a thermal diffusivity D. One side of the measurement sample 1, or the first surface 103, is uniformly and periodically heated by a laser beam L1 at an angular frequency $\omega$. The first surface 103 in this example is subjected to area heating by the laser beam L1.

The symbols used in the following descriptions are as follows:

   p: absorbed power per unit area (W/m$^2$)
   k: thermal conductivity (W/m·K)
   D: thermal diffusivity (m$^2$/s)
   d: thickness (m)
   $\omega$: angular frequency (1/s)

The temperature of the second surface 105, which is the other side of the heated measurement sample 1, is expressed by equation (2).

[Equation 2]

$$\tilde{T}(z,t) = \frac{ip}{2\pi\kappa\lambda\sinh(\lambda d)}\cosh\big(\lambda(d-z)\big)e^{i\omega t} \qquad \cdots(2)$$

$\lambda$ is a complex wavenumber, which is expressed by equation (3).

[Equation 3]

$$\lambda = \sqrt{\frac{i\omega}{D}} \qquad\qquad\qquad \cdots(3)$$

The temperature amplitude A is expressed by equation (4) from the absolute value of the temperature T.

[Equation 4]

$$A(z) = \big|\tilde{T}(z)\big| = \left|\frac{ip}{2\pi\kappa\lambda\sinh(\lambda d)}\cosh\big(\lambda(d-z)\big)\right| \qquad \cdots(4)$$

The phase lag obtained from the argument of the temperature T is expressed by equation (5).

[Equation 5]

$$\varphi(z) = \arg\left|\tilde{T}(z)\right| = \arg\left|\frac{ip}{2\pi\kappa\lambda\sinh(\lambda d)}\cosh(\lambda(d-z))\right| \quad \cdots(5)$$

Hence, the relationship of equation (6) holds.
[Equation 6]

$$\frac{\partial A}{\partial p}(z) = \left|\frac{i}{2\pi\kappa\lambda\sinh(\lambda d)}\cosh(\lambda(d-z))\right| \quad \cdots(6)$$

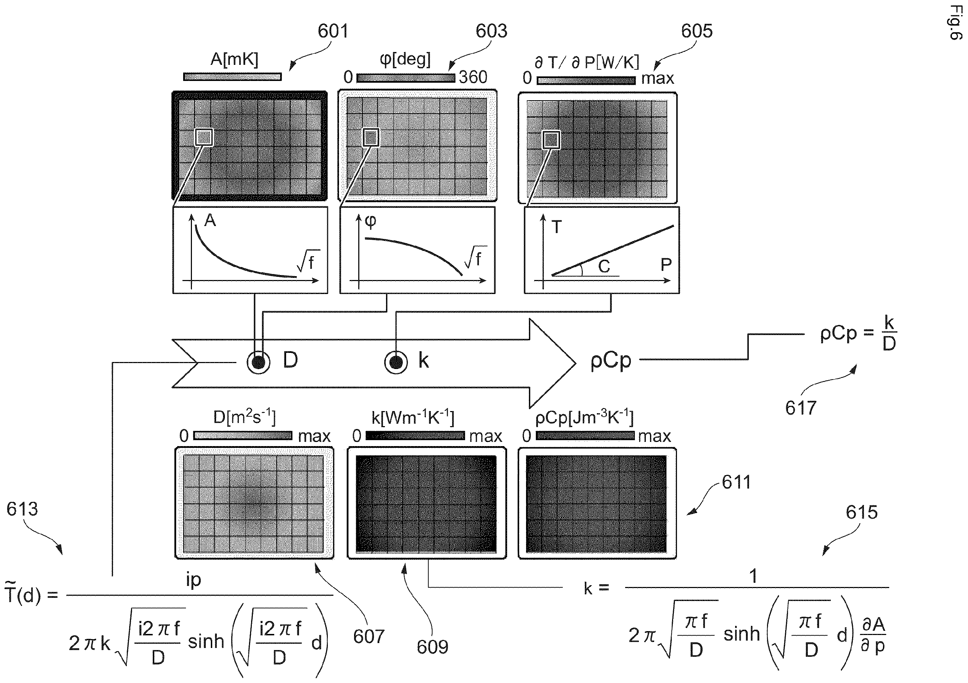

FIG. 6 illustrates the principle of measuring a specific heat capacity in the first embodiment.
Referring now to FIG. 6, the principle of measuring a specific heat capacity in the present embodiment is described.
First, a through-plane thermal diffusivity D is obtained by measuring the temperature amplitude (see reference numeral 601) and the phase lag (see reference numeral 603) while varying the frequency and then fitting the temperature amplitude of the above equation (4) and the phase lag of the above equation (5) to the obtained curves (see reference numerals 607, 613 in the figure).
A through-plane thermal conductivity k is obtained from the linear slope of laser power dependence of the temperature amplitude (see reference numerals 609, 615), which is obtained by measuring the temperature amplitude (see reference numeral 605) while varying the laser power but keeping the frequency constant. To further illustrate, in the present embodiment, a distribution of the through-plane thermal conductivity k is output as an image (see reference numeral 609 in the figure).
Then, a specific heat capacity pCp is calculated from the above through-plane thermal diffusivity D and through-plane thermal conductivity k based on the above equation (1) (see reference numerals 611, 617). To further illustrate, in the present embodiment, a distribution of the specific heat capacity pCp is output as an image (see reference numeral 611).

<Fatigue Evaluation Operation>

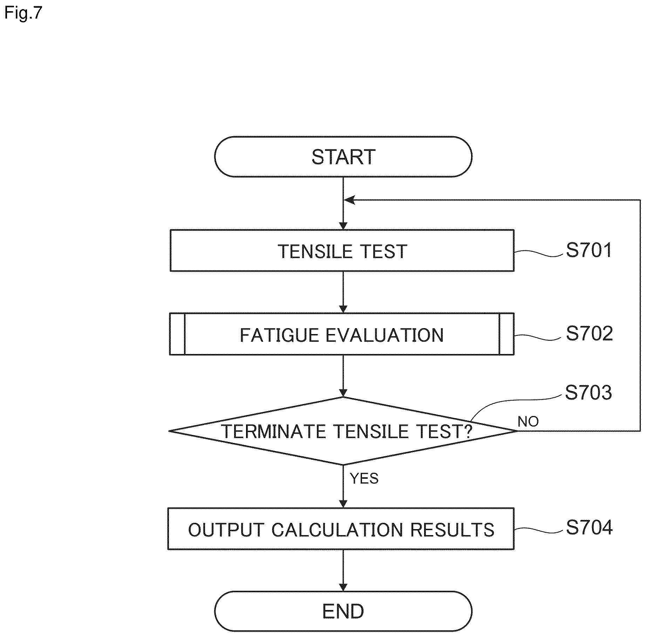

FIG.7 is a flowchart of a fatigue evaluation and determination operation by the thermophysical property evaluation device 100 (see FIG. 1).
Referring now to FIGS. 1 and 7, a fatigue evaluation operation by the thermophysical property evaluation device 100 of the present embodiment is described. In this example, a tensile load is repeatedly applied to the measurement sample 1 by a tensile tester (not shown). In response to the number of load cycles reaching a predetermined count, the thermophysical property evaluation device 100 performs the fatigue evaluation operation. For example, the fatigue evaluation operation is performed in response to the number of load cycles reaching 1,000, 10,000, and 100,000. It is assumed that the fatigue condition of the measurement sample 1 in an undamaged condition, i.e., at zero load cycles, has already been measured.
As shown in FIG. 7, in the fatigue evaluation operation, a tensile test is first performed by a tensile tester (not shown) (S701). After the tensile test has been performed a predetermined number of times, the thermophysical property evaluation device 100 performs the fatigue evaluation (S702).
The fatigue evaluation calculation unit 58 determines whether to terminate the tensile test, i.e., whether the number of load cycles has reached a set value (S703). If the tensile test is to be terminated (YES in S703), the calculation result output unit 59 outputs the calculation results for the measurement sample 1 to a display area (not shown) such as an LCD display (S704). On the other hand, if the tensile test is not to be terminated (NO in S703), the tensile test is performed by the tensile tester (S701).

<Fatigue Evaluation Process>

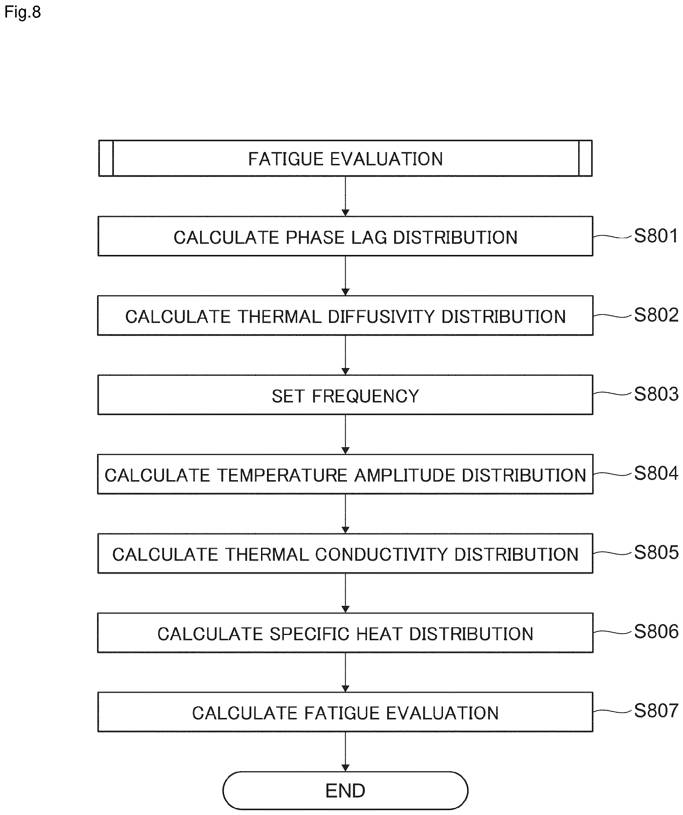

FIG.8 is a flowchart of a fatigue evaluation process by the thermophysical property evaluation device 100 (see FIG. 1).
Referring now to FIGS. 1 and 8, a fatigue evaluation process by the thermophysical property evaluation device 100 in the present embodiment is described.
First, the phase lag distribution calculation unit 53 calculates the phase lag distribution (S801). Specifically, the measurement sample 1 is periodically heated by the laser beam emitted from the diode laser 10. The phase lag distribution calculation unit 53 obtains the temperature distribution via the infrared thermographic camera 40 while varying the frequency of the laser beam emitted from the diode laser 10, and calculates the phase lag distribution. Based on the calculated phase lag distribution, the thermal diffusivity distribution calculation unit 55 calculates the thermal diffusivity distribution (S802).

Then, the temperature amplitude distribution calculation unit 54 sets the frequency to be used in measuring the temperature amplitude distribution (S803).

The temperature amplitude distribution calculation unit 54 then calculates the temperature amplitude distribution (S804). Specifically, the measurement sample 1 is periodically heated by the laser beam emitted from the diode laser 10. The temperature amplitude distribution calculation unit 54 obtains the temperature distribution via the infrared thermographic camera 40 while varying the laser power of the laser beam emitted from the diode laser 10, and calculates the temperature amplitude distribution. Based on the calculated temperature amplitude distribution, the thermal conductivity calculation unit 56 calculates the thermal conductivity distribution (S805).

Then, the specific heat calculation unit 57 calculates the specific heat distribution (S806). Based on the calculated specific heat distribution, the fatigue evaluation calculation unit 58 calculates the fatigue evaluation of the measurement sample 1 (S807).

Depending on the physical property values of the measurement sample 1, appropriate frequencies for the temperature amplitude measurement by the temperature amplitude distribution calculation unit 54 vary. Hence, in the illustrated example, the phase lag distribution calculation unit 53 calculates the phase lag distribution prior to the temperature amplitude measurement by the temperature amplitude distribution calculation unit 54. Then, within the range of frequencies set by the phase lag distribution calculation unit 53, the frequencies for the temperature amplitude measurement are set (S803). This enables a more efficient temperature amplitude measurement by the temperature amplitude distribution calculation unit 54.

To further illustrate, the order of performance of the phase lag measurement by the phase lag distribution calculation unit 53 and the temperature amplitude measurement by the temperature amplitude distribution calculation unit 54 is not limited. For example, when the physical property values of the measurement sample 1 are known, the temperature amplitude distribution calculation unit 54 may measure the temperature amplitude prior to the phase lag measurement by the phase lag distribution calculation unit 53.

<Measurement Results 1>

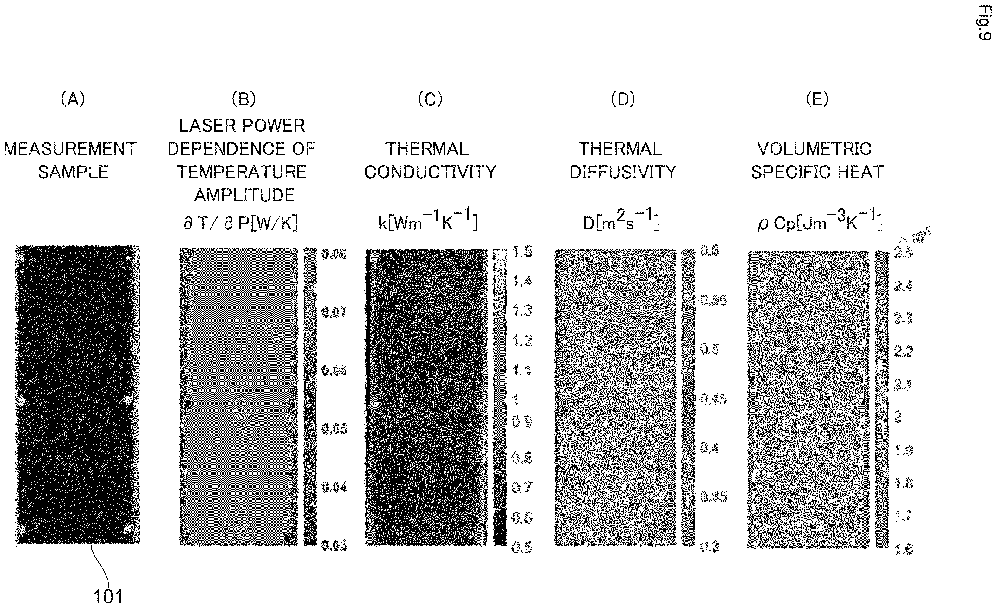

FIGS. 9(A) through 9(E) illustrate first measurement results. Specifically, FIG. 9(A) illustrates a first sample 101, which is an example of the measurement sample 1. FIG. 9(B) illustrates a distribution of laser power dependence of the temperature amplitude. FIG. 9(C) illustrates a thermal conductivity distribution. FIG. 9(D) illustrates a thermal diffusivity distribution. FIG. 9(E) illustrates a volumetric specific heat distribution.

Referring now to FIG. 9, the first measurement results using the first sample 101 are described. This first sample 101 is a 36 mm wide, 120 mm long, and 1 mm thick composite material, specifically a carbon fiber-reinforced resin. The region of observation in the first sample 101 is 36 mm wide and 45 mm long.

The frequency of the laser used for the phase lag distribution calculation (first measurement; see S801 in FIG. 8 above) is varied in units of 0.02 Hz from 0.3 to 0.54 Hz. This laser has a laser power of 1750 mW. Also, the laser power of the laser used for the temperature amplitude distribution calculation (second measurement; see S804 in FIG. 8 above) is varied in units of 250 mW from 500 to 2000 mW. This laser has a frequency of 0.125 Hz. As used herein, varying the laser power means switching the maximum output for one period of the laser beam with which the periodic heating is performed. In other words, varying the laser power means switching the maximum output of the laser beam at least between a first maximum output and a second maximum output. In still other words, varying the laser power means varying the irradiation amplitude of the laser beam.

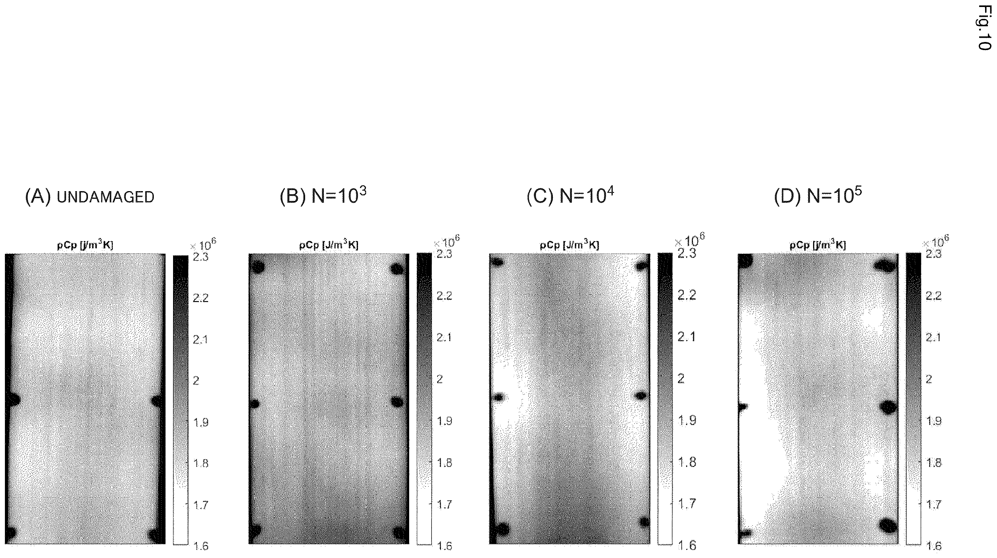

As shown in FIGS. 9(B) through 9(E), the distribution of laser power dependence of the temperature amplitude, the thermal conductivity distribution, the thermal diffusivity distribution, and the volumetric specific heat distribution of the first sample 101 were calculated. This indicates that the specific heat of the first sample 101 can be measured in a nondestructive and non-contact manner. To further illustrate, FIGS. 10(B) through 10(E) indicate that the specific heat distribution of the first sample 101 can be detected by the thermophysical property evaluation device 100 (see FIG. 1).

<Measurement Results 2>

FIGS. 10(A) through 10(D) illustrate second measurement results. Specifically, FIGS. 10(A) through 10(D) illustrate results of evaluation of the progress of fatigue degradation with increased number of load cycles, as evaluated based on specific heat. To further illustrate, FIG. 10(A) illustrates a volumetric specific heat distribution of the above first sample 101 (see FIG. 9) in an undamaged condition, i.e., at zero load cycles (N). Similarly, FIGS. 10(B), 10(C), and 10(D) illustrate volumetric specific heat distributions at $10^3$, $10^4$, and $10^5$ load cycles, respectively. As shown in FIGS. 10(A) through 10(D), the volumetric specific heat distribution of the first sample 101 was found to vary with an increase in the number of load cycles.

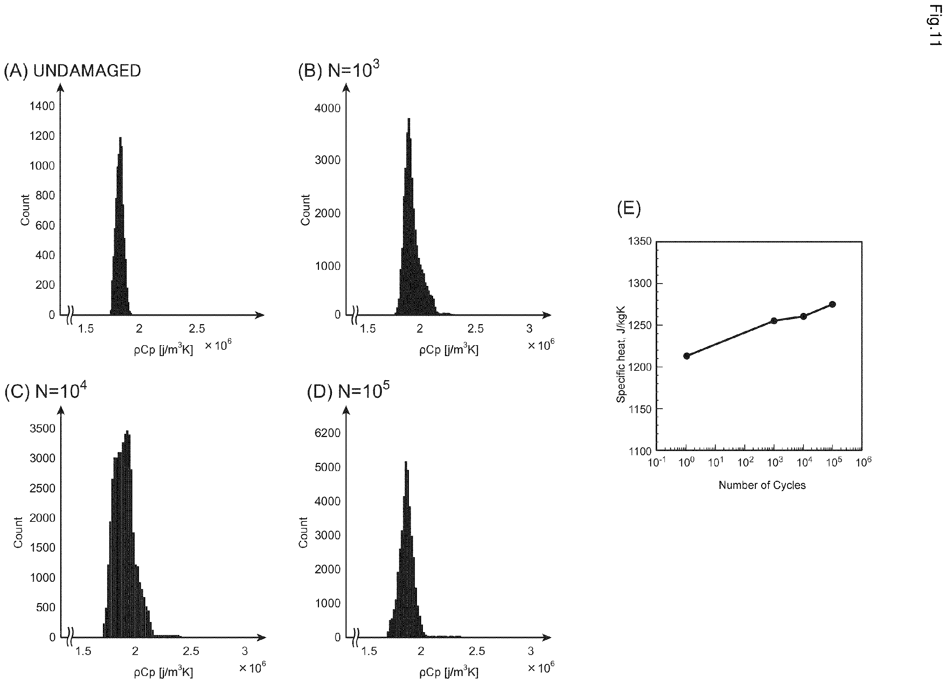

FIGS. 11(A) through 11(E) illustrate the second measurement results. Specifically, FIGS. 11(A) through 11(D) illustrate changes in specific heat calculated from the specific heat distributions respectively shown in FIGS. 10(A) through 10(D) above. More specifically, the horizontal and vertical axes in FIGS. 11(A) through 11(D) represent the volumetric specific heat and the count, respectively. FIG. 11(E) illustrates changes in specific heat capacity as a function of the number of load cycles.

As shown in FIGS. 11(A) through 11(D), the specific heat was observed to vary with an increase in the number of load cycles. Also, as shown in FIG. 11(E), the specific heat, or entropy, was observed to tend to increase with an increase in the number of load cycles. More specifically, FIGS. 11(A) through 11(E) indicate that the progress of fatigue degradation of the first sample 101 can be measured by the thermophysical property evaluation device 100 (see FIG. 1) based on specific heat.

As described above, the thermophysical property evaluation device 100 (see FIG. 1) can measure the thermal conductivity in a nondestructive and non-contact manner. To further illustrate, for example, the thermophysical property evaluation device 100 can measure in-situ the thermal conductivity of a measurement sample 1 that has already been finished as a product. To further illustrate, the thermophysical property evaluation device 100 can measure the thermal conductivity of even a rare measurement sample 1 in a nondestructive manner.

One well-known technique that has been used to measure specific heat is a differential scanning calorimetry (DSC) method. However, the DSC method requires destructive testing, i.e., cutting and crushing of samples, and possible structural changes during crushing processes may cause measurements to vary. In addition, the DSC method requires a reference material whose specific heat value is known. Moreover, while the DSC method can obtain an average specific heat value of a sample, it is difficult for this method to measure its specific heat distribution.

In contrast, the thermophysical property evaluation device 100 (see FIG. 1) can measure in-situ the specific heat in a nondestructive and non-contact manner. In addition, the thermophysical property evaluation device 100 can do without reference materials for specific heat measurement, improving the reliability of the measurement. Moreover, the thermophysical property evaluation device 100 can measure a specific heat distribution, so that it can detect areas with different physical properties in the measurement sample 1 (i.e., can detect damages).

<Second Embodiment>

<Configuration of Thermophysical Property Evaluation Device 200>

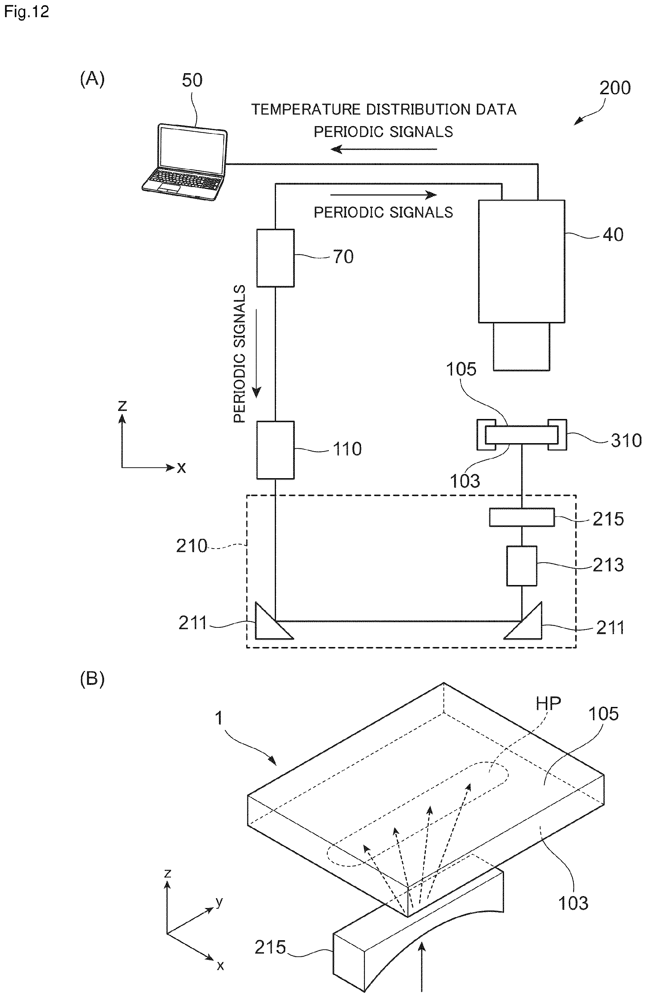

FIG. 12 illustrates a schematic configuration of a thermophysical property evaluation device 200 according to a second embodiment.

Referring now to FIG. 12, a configuration of the thermophysical property evaluation device 200 according to the second embodiment is described. Similar components to those in the above embodiment are hereinafter identified by the same reference numerals, and detailed descriptions thereof may be omitted.

While the above description discussed performing the area heating on the first surface 103 of the measurement sample 1, this is not limiting. For example, line heating may be performed on the first surface 103 of the measurement sample 1, as in the thermophysical property evaluation device 200 shown in FIG. 12.

Specifically, as shown in FIG. 12(A), the thermophysical property evaluation device 200 according to the present embodiment includes a diode laser 110 serving as a light source for heating the measurement sample 1, a light guide 210 for directing a laser beam of the diode laser 110 to the measurement sample 1, and a holder 310 for holding the measurement sample 1.

As shown in FIG. 12(B), the diode laser 110 heats a portion (heating region Hp) of the measurement sample 1 that extends in one direction on the surface thereof. In other words, the diode laser 110 is a light source for line heating.

As shown in FIG. 12(A), the light guide 210 includes a mirror 211 to reflect a laser beam emitted from the diode laser 110, a beam expander 213 to expand the beam diameter of the laser beam from the mirror 211, and a cylindrical lens 215 to shape the laser beam from the beam expander 213 into sheet light.

In the thermophysical property evaluation device 200, the laser beam emitted from the diode laser 110 is incident on the measurement sample 1 through the mirror 211, the beam expander 213, and the cylindrical lens 215. To further illustrate, the laser beam emitted from the diode laser 110 is incident in a direction perpendicular to the first surface 103 of the measurement sample 1. In this measurement sample 1, the region irradiated with the laser beam (region extending in one direction) is periodically heated. In other words, a specific region (heating region Hp) in the first surface 103 of the measurement sample 1, or a spot, is periodically heated.

The computer 50, in combination with the infrared thermographic camera 40, continuously captures infrared images and performs computations at predetermined frame rate intervals to create an averaged image based on the amount of temperature change over time (lock-in method). To further illustrate, the data obtained from the infrared thermographic camera 40 is processed by the computer 50 to calculate an in-plane thermal conductivity of the measurement sample 1. The data obtained from the infrared thermographic camera 40 is also processed by the computer 50 to calculate information about thermophysical properties of the measurement sample 1.

The thermophysical property evaluation device 100 in the above first embodiment uses the diode laser 10 and the light guide 20 to emit light with a uniform intensity distribution onto the surface of the measurement sample 1. On the other hand, the thermophysical property evaluation device 200 does not require an arrangement to homogenize the light.

<In-Plane Specific Heat Distribution>

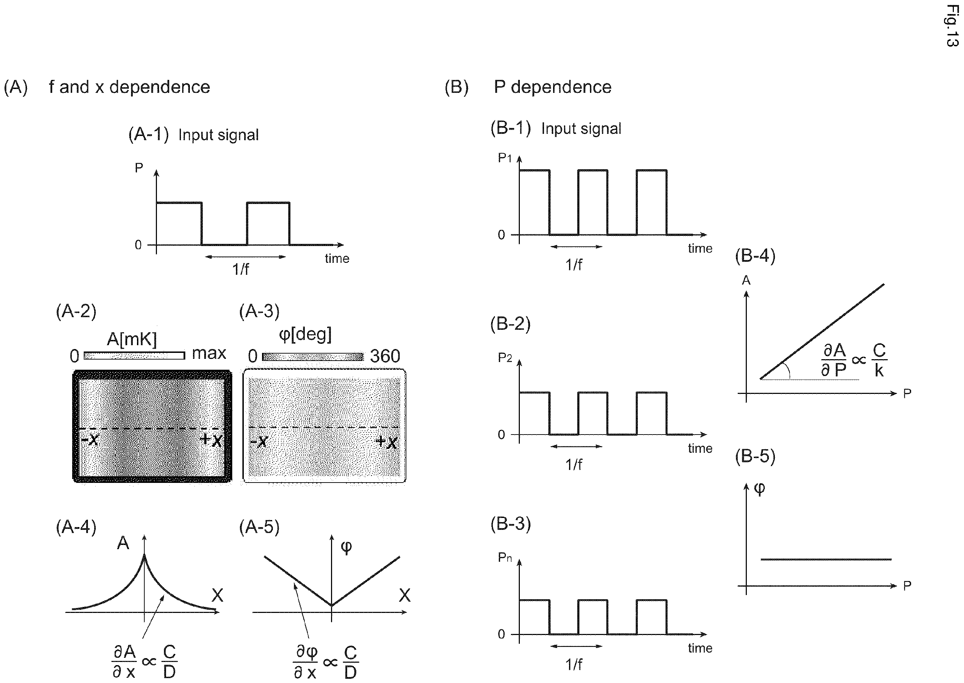

FIGS. 13(A) and 13(B) illustrate the principles of measuring thermophysical properties in the second embodiment, where FIG. 13(A) illustrates the principle of measuring a thermal diffusivity, and FIG. 13(B) illustrates the principle of measuring a thermal conductivity.

The measurement principles in the second embodiment are now outlined with reference to FIGS. 13(A) and 13(B). Here we discuss measuring a specific heat distribution of the measurement sample 1 in the in-plane direction.

First, as shown in FIG. 13(A), a thermal diffusivity is measured using the thermophysical property evaluation device 200. Specifically, a distance dependence of the temperature amplitude (see FIGS. 13(A-2) and 13(A-3)) and a distance dependence of the phase lag (see FIGS. 13(A-4) and 13(A-5)) are measured by periodically heating the measurement sample 1 with the laser beam emitted from the diode laser 10 while keeping its power and frequency constant as shown in FIG. 13(A-1). A thermal diffusivity distribution is then calculated from the measured distance dependences of the temperature amplitude and the phase lag.

Also, a power dependence of the temperature amplitude (see FIG. 13(B-4)) is measured by periodically heating the measurement sample 1 with the laser beam emitted from the diode laser 10 while varying its power but keeping its frequency constant as shown in FIGS. 13(B-1) through 13(B-3). A thermal conductivity distribution is then calculated from the measured power dependence of the temperature amplitude. In other words, in the present embodiment, the thermal conductivity distribution is determined from the relationship between the temperature amplitude decay and the laser power.

Then, using the measured thermal diffusivity distribution and thermal conductivity distribution as well as a density distribution measured by a well-known technique, a specific heat distribution is calculated from the above equation (1).

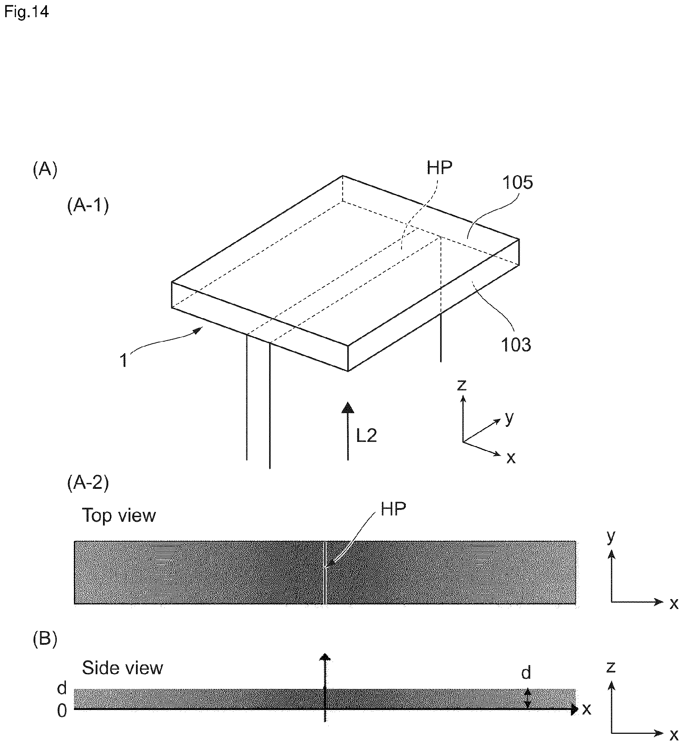

FIGS. 14(A) and 14(B) illustrate a model of the measurement principles in the second embodiment, where the figures depict heating conditions.

The measurement principles in the present embodiment are now detailed with reference to FIGS. 14(A) and 14(B).

As shown in FIGS. 14(A) and 14(B), the generally planar measurement sample 1 has a thickness d. The measurement sample 1 shall be a thermally thin object. This measurement sample 1 has a thermal conductivity k and a thermal diffusivity D. One side of the measurement sample 1, or the first surface 103, is uniformly and periodically heated by a laser beam L2 at an angular frequency $\omega$. The first surface 103 in this example is subjected to line heating by the laser beam L2. The region heated by the laser beam L2 on the first surface 103, or a heating region Hp, is a band-shaped region extending along the y direction at x = 0.

The symbols used in the following descriptions are as follows:

p: absorbed power per unit area (W/m$^2$)
k: thermal conductivity (W/m·K)
D: thermal diffusivity (m$^2$/s)
d: thickness (m)
$\omega = 2\pi f$ (rad/s)

First, the temperature at a distance x from the heating region Hp on the other side of the heated measurement sample 1, or the second surface 105, is expressed by equation (7).
[Equation 7]

$$\tilde{T}(x, t) = \frac{2P}{\pi \kappa d} \sqrt{\frac{D}{\omega}} e^{\pm \sqrt{\frac{\omega}{2D}} x + i \left( n\omega t \mp \sqrt{\frac{\omega}{2D}} x - \frac{\pi}{4} \right)} \qquad \cdots (7)$$

The temperature amplitude A is expressed by equation (8).
[Equation 8]

$$A(x) = \frac{2P}{\pi\kappa d}\sqrt{\frac{D}{\omega}}\, e^{\mp\sqrt{\frac{\omega}{2D}}x} \qquad\qquad \cdots(8)$$

The phase lag is expressed by equation (9).
[Equation 9]

$$\varphi(x) = \mp\sqrt{\frac{\omega}{2D}}\,x + \frac{\pi}{4} \qquad\qquad \cdots(9)$$

Hence, the relationship of equation (10) holds.
[Equation 10]

$$\frac{\partial A}{\partial P}(x) = \frac{2}{\pi\kappa d}\sqrt{\frac{D}{\omega}}\, e^{\mp\sqrt{\frac{\omega}{2D}}x} \qquad\qquad \cdots(10)$$

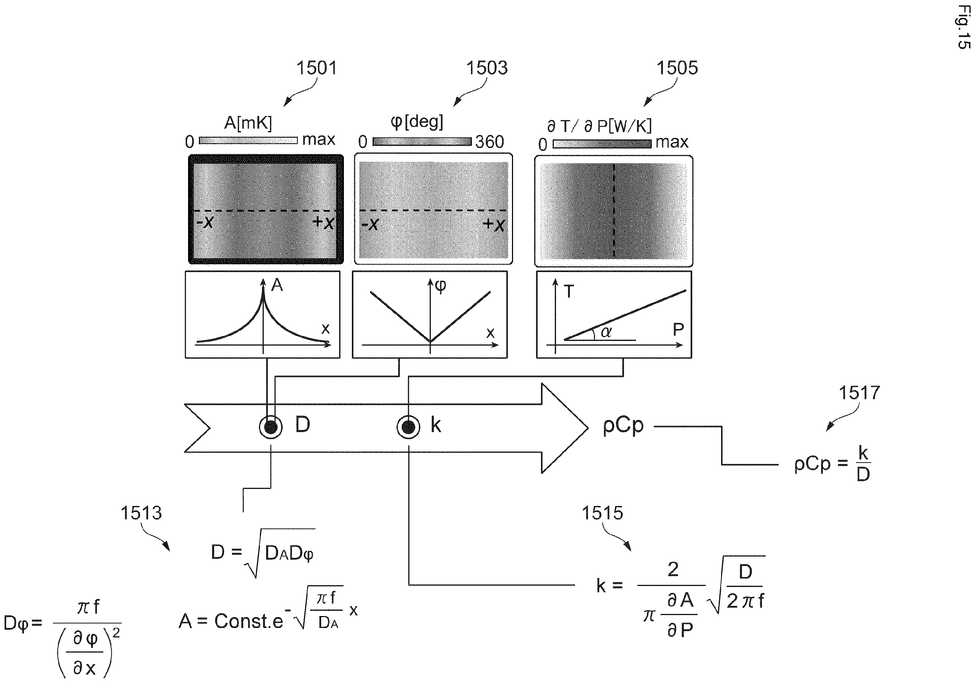

FIG. 15 illustrates the principle of measuring a specific heat capacity in the second embodiment.

Referring now to FIG. 15, the principle of measuring a specific heat capacity in the present embodiment is described.

First, an in-plane thermal diffusivity D is obtained by measuring the distance dependence of the temperature amplitude (see reference numeral 1501) and the distance dependence of the phase lag (see reference numeral 1503) and then fitting the temperature amplitude of the above equation (8) and the phase lag of the above equation (9) to the obtained curves (see reference numeral 1513 in the figure).

An in-plane thermal conductivity k is obtained from the linear slope of laser power dependence of the temperature amplitude (see reference numeral 1515), which is obtained by measuring the temperature amplitude (see reference numeral 1505).

Then, a specific heat capacity pCp is calculated from the above in-plane thermal diffusivity D and in-plane thermal conductivity k based on the above equation (1) (see reference numeral 1517).

<Measurement Results 3>

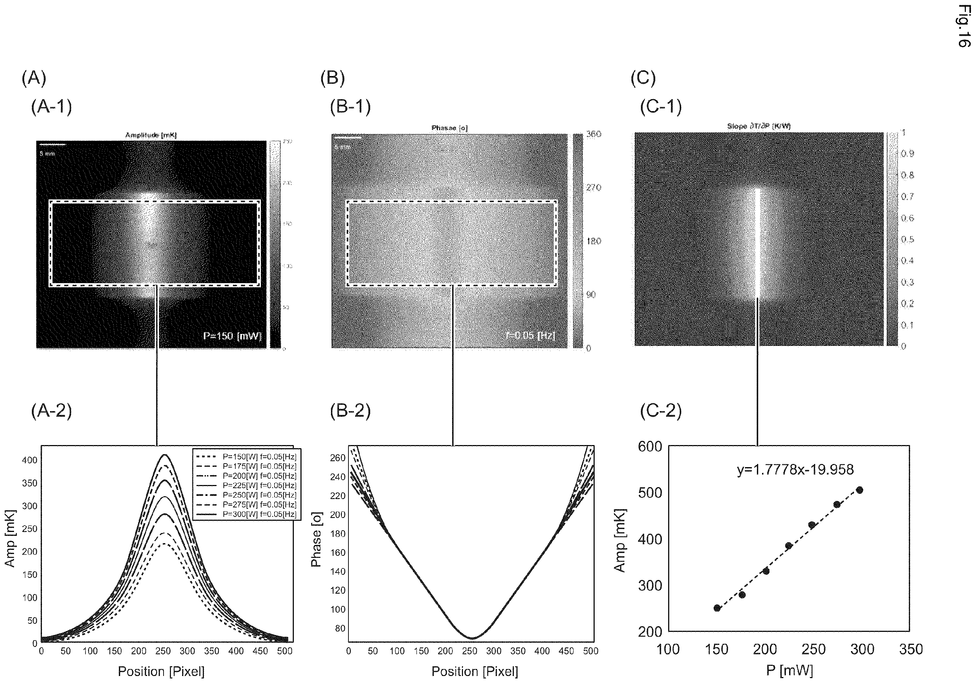

FIGS. 16(A) through 16(C) illustrate third measurement results. Specifically, FIG. 16(A) illustrates the laser power dependence of the temperature amplitude. FIG. 16(B) illustrates the laser power dependence of the phase. FIG. 16(C) illustrates the laser power dependence of the temperature amplitude.

The third measurement results are now shown with reference to FIG. 16. The measurement sample 1 in this measurement is a composite material. The frequency of the laser used for the phase lag distribution calculation (see S801 in FIG. 8 above) is varied in units of 0.02 Hz from 0.3 to 0.54 Hz. This laser has a laser power of 150 mW. Also, the laser power of the laser used for the temperature amplitude distribution calculation (see S804 in FIG. 8 above) is varied in units of 25 mW from 150 to 300 mW. This laser has a frequency of 0.05 Hz.

As shown in FIG. 16(A), the temperature amplitude was found to be dependent on the laser power. On the other hand, as shown in FIG. 16(B), the phase was observed to be independent of the laser power. The thermophysical properties calculated from FIG. 16(A) through 16(C) were as follows: the in-plane thermal diffusivity was 1.06 ($m^2$/s), the in-plane thermal conductivity was 1.84 (W/m·K), and the specific heat capacity was 1641.1 (kJ/$m^3$K).

<Third Embodiment>

<Configuration of Thermophysical Property Evaluation Device 300>

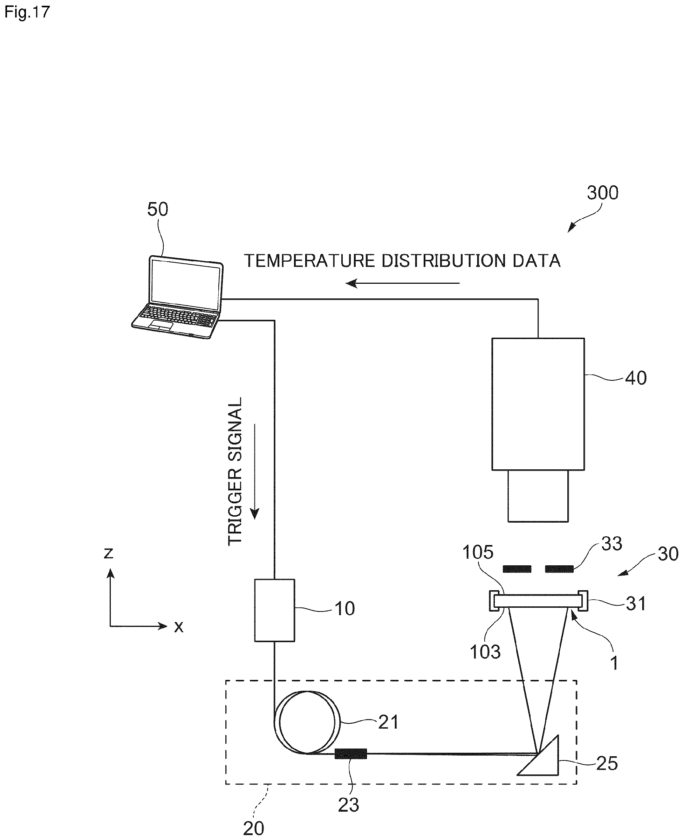

FIG. 17 illustrates a schematic configuration of a thermophysical property evaluation device 300 according to a third embodiment.

Referring now to FIG. 17, a configuration of the thermophysical property evaluation device 300 according to the third embodiment is described. Similar components to those in the above embodiments are hereinafter identified by the same reference numerals, and detailed descriptions thereof may be omitted.

While the above description discussed creating an averaged image from the amount of temperature change over time (lock-in method), this is not limiting. For example, a high-intensity, short-duration uniform laser beam may be emitted once, i.e., pulsed, onto the first surface 103 of the measurement sample 1, as in the thermophysical property evaluation device 300 shown in FIG. 17.

Specifically, the thermophysical property evaluation device 300 according to the present embodiment includes the diode laser 10 serving as a light source for heating the measurement sample 1, the light guide 20 for directing the laser beam of the diode laser 10 to the measurement sample 1, a holder 310 for holding the measurement sample 1, the infrared thermographic camera 40, and the computer 50. The thermophysical property evaluation device 300 does not include the periodic signal generator 70 (see FIG. 1).

While the above description in relation to FIG. 1 discussed performing the periodic heating, the thermophysical property evaluation device 300 emits a high-intensity, short-duration uniform laser pulse once to heat the surface of the measurement sample 1.

<Through-Plane Specific Heat Distribution>

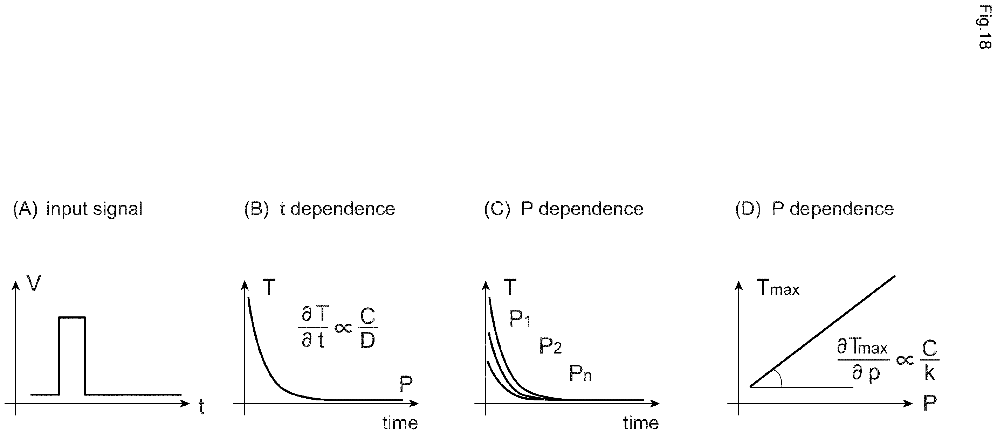

FIGS. 18(A) through 18(D) illustrate the principles of measuring thermophysical properties in the third embodiment, where FIG. 18(A) illustrates a heating laser, FIG. 18(B) illustrates the principle of measuring a thermal diffusivity, and FIGS. 18(C) and 18(D) illustrate the principle of measuring a thermal conductivity.

The measurement principles in the third embodiment are now outlined with reference to FIGS. 18(A) through 18(D). Here we discuss measuring a specific heat distribution of the measurement sample 1 in the through-plane direction.

First, the measurement sample 1 is heated by a pulsed laser from the diode laser 10, as shown in FIG. 18(A). This heating is used to measure a time dependence of the temperature (see FIG. 13(B)), i.e., temperature decay. A thermal diffusivity distribution is then calculated from the measured time dependence of the temperature.

Then, as shown in FIG. 18(C), a power dependence of the maximum temperature is measured by heating the measurement sample 1 with the laser beam emitted from the diode laser 10 while varying its power. Then, as shown in FIG. 18(D), a thermal conductivity distribution is calculated from the measured power dependence of the maximum temperature. In other words, in the present embodiment, the thermal conductivity distribution is determined from the relationship between the maximum temperature and the laser power.

Then, using the measured thermal diffusivity distribution and thermal conductivity distribution as well as a density distribution measured by a well-known technique, a specific heat distribution is calculated from the above equation (1).

The measurement principles in the present embodiment are now detailed.

Here, the measurement principles are discussed with reference to the generally planar measurement sample 1 as shown in FIGS. 5(A) and 5(B) above. This measurement sample 1 has a thickness d. Also, the measurement sample 1 shall have a thermal conductivity k and a thermal diffusivity D. One side of the measurement sample 1, or the first surface 103, is uniformly heated by the laser beam L1. The first surface 103 in this example is subjected to area heating by the laser beam L1.

The symbols used in the following descriptions are as follows:

p: absorbed power per unit area (W/m$^2$)
k: thermal conductivity (W/m·K)
D: thermal diffusivity (m$^2$/s)
d: thickness (m)
t: time (s)

The temperature of the other side of the heated measurement sample 1, or the second surface 105, is expressed by equation (11).

[Equation 11]

$$T(t) = \frac{Dp}{\kappa d}\left[1 + 2\sum_{n=1}^{\infty}(-1)^n \exp\left(-\frac{n^2\pi^2 D}{d^2}t\right)\right] \qquad \cdots(11)$$

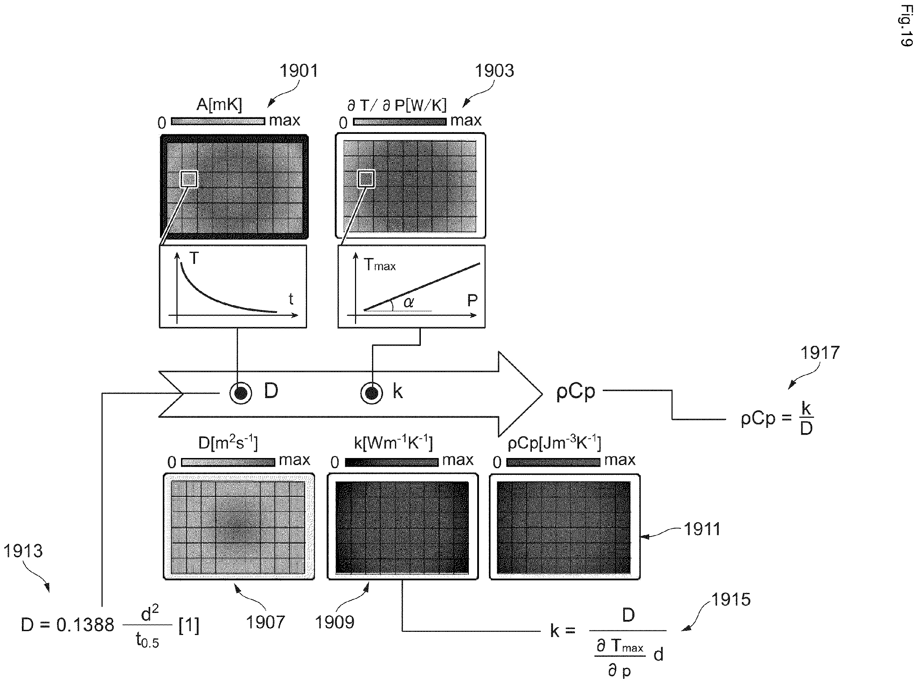

FIG. 19 illustrates the principle of measuring a specific heat capacity in the third embodiment.

Referring now to FIG. 19, the principle of measuring a specific heat capacity in the present embodiment is described.

First, a through-plane thermal diffusivity D is obtained by measuring a time dependence of the temperature, i.e., exponential decay of the temperature (see reference numeral 1901), and fitting, for example, a well-known formula (see reference numeral 1913) to the obtained curve (see reference numeral 1907 in the figure). In the illustrated formula, time $t_{0.5}$ until the temperature rise reaches 50% of its maximum value is defined as an exponential decay to calculate the thermal diffusivity D.

A through-plane thermal conductivity k is obtained from the linear slope of laser power dependence of the maximum temperature (see reference numerals 1909, 1915 in the figure), which is obtained by measuring the maximum temperature (see reference numeral 1903) at each pixel (at each location).

Then, a specific heat capacity pCp is calculated from the above through-plane thermal diffusivity D and through-plane thermal conductivity k based on the above equation (1) (see reference numerals 1911, 1917).

<Fourth Embodiment>

<Configuration of Thermophysical Property Evaluation Device 400>

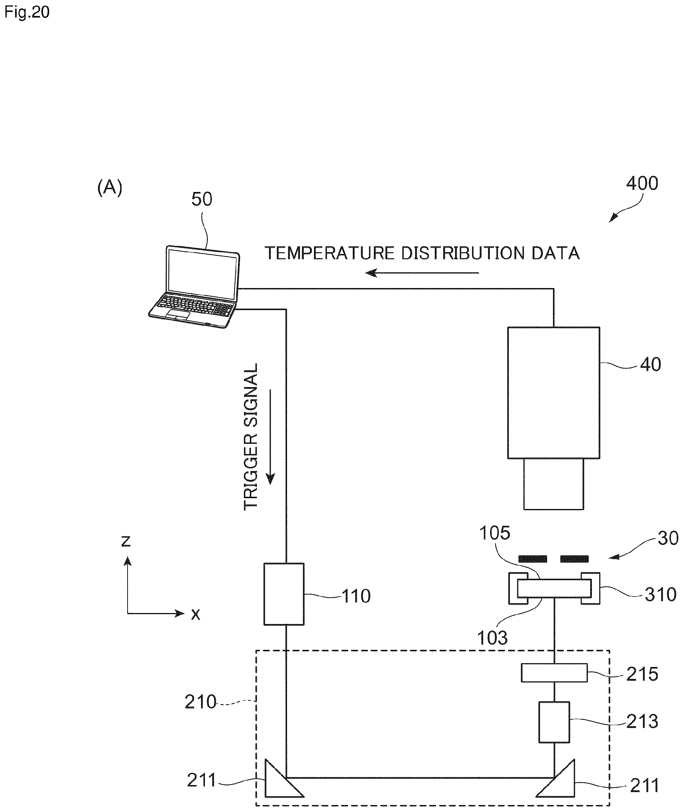

FIG. 20 illustrates a schematic configuration of a thermophysical property evaluation device 400 according to a fourth embodiment.

Referring now to FIG. 20, a configuration of the thermophysical property evaluation device 400 according to the fourth embodiment is described. Similar components to those in the above embodiments are hereinafter identified by the same reference numerals, and detailed descriptions thereof may be omitted.

The thermophysical property evaluation device 400 shown in FIG. 20 may be configured to emit a pulsed laser beam onto a region of the measurement sample 1 extending in one direction on the first surface 103 thereof.

Specifically, the thermophysical property evaluation device 400 according to the present embodiment includes the diode laser 110 serving as a light source for heating the measurement sample 1, the light guide 210 for directing the laser beam of the diode laser 110 to the measurement sample 1, a holder 310 for holding the measurement sample 1, the infrared thermographic camera 40, and the computer 50. The thermophysical property evaluation device 400 does not include the periodic signal generator 70 (see FIG. 1).

While the above description in relation to FIG. 12 discussed performing the periodic heating, the thermophysical property evaluation device 400 emits a high-intensity, short-duration uniform laser pulse once to heat the surface of the measurement sample 1.

<In-Plane Specific Heat Distribution>

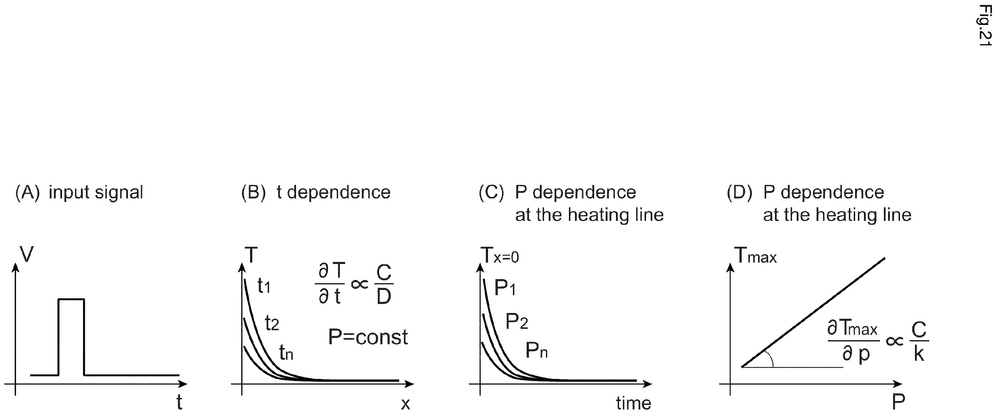

FIGS. 21(A) through 21(D) illustrate the principles of measuring thermophysical properties in the fourth embodiment, where FIG. 21(A) illustrates a heating laser, FIG. 21(B) illustrates the principle of measuring a thermal diffusivity, and FIGS. 21(C) and 21(D) illustrate the principle of measuring a thermal conductivity.

The measurement principles in the fourth embodiment are now outlined with reference to FIGS. 21(A) through 21(D). Here we discuss measuring a specific heat distribution of the measurement sample 1 in the in-plane direction.

First, the measurement sample 1 is heated by a pulsed laser from the diode laser 110, as shown in FIG. 21(A). This heating is used to measure a time dependence of the temperature (see FIG. 21(B)), i.e., temperature decay. A thermal diffusivity distribution is then calculated from the measured time dependence of the temperature.

Then, as shown in FIG. 21(C), a power dependence of the maximum temperature is measured by heating the measurement sample 1 with the laser beam emitted from the diode laser 110 while varying its power. Then, as shown in FIG. 21(D), a thermal conductivity distribution is calculated from the measured power dependence of the maximum temperature. In other words, in the present embodiment, the thermal conductivity distribution is determined from the relationship between the maximum temperature and the laser power.

Then, using the measured thermal diffusivity distribution and thermal conductivity distribution as well as a density distribution measured by a well-known technique, a specific heat distribution is calculated from the above equation (1).

The measurement principles in the present embodiment are now detailed.

Here, the measurement principles are discussed with reference to the generally planar measurement sample 1 as shown in FIGS. 14(A) and 14(B) above. This measurement sample 1 has a thickness d. Also, the measurement sample 1 shall have a thermal conductivity k and a thermal diffusivity D. One side of the measurement sample 1, or the first surface 103, is heated by the laser beam L2. The first surface 103 in this example is subjected to line heating by the laser beam L2.

The symbols used in the following descriptions are as follows:

p: absorbed power per unit area (W/m$^2$)

k: thermal conductivity (W/m·K)
D: thermal diffusivity (m²/s)
d: thickness (m)
t: half the width of the heating region

The temperature of the other side of the heated measurement sample 1, or the second surface 105, is expressed by equation (12).
[Equation 12]

$$T(x, Dt) = \frac{1}{\sqrt{\pi}} \int_{-\infty}^{+\infty} T_o\left(x + 2\beta\sqrt{Dt}, 0\right) e^{-\beta^2} \mathrm{d}\beta \qquad \cdots(12)$$

The initial condition is expressed by equation (13).
[Equation 13]

$$T_o(x, 0) = \begin{cases} \dfrac{Dp}{\kappa} \ (-l < x < l) \\ 0 \ (x \leq -l \ or \ l \leq x) \end{cases} \qquad \cdots(13)$$

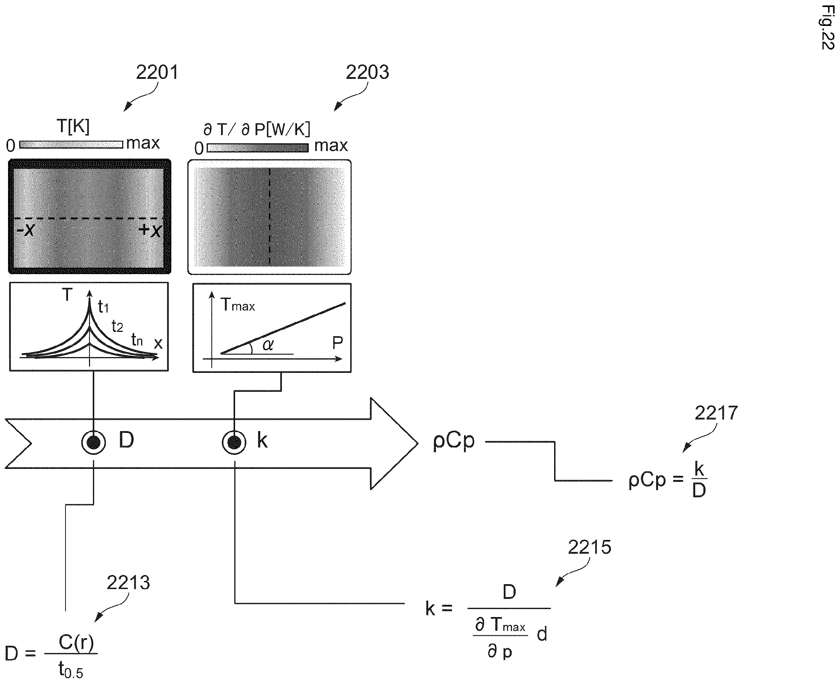

FIG. 22 illustrates the principle of measuring a specific heat capacity.
Referring now to FIG. 22, the principle of measuring a specific heat capacity in the present embodiment is described.
First, an in-plane thermal diffusivity D is obtained by measuring a time dependence of the temperature, i.e., exponential decay of the temperature (see reference numeral 2201), and fitting, for example, a well-known formula (see reference numeral 2213) to the obtained curve. In the illustrated formula, time $t_{0.5}$ until the temperature rise reaches 50% of its maximum value is defined as an exponential decay to calculate the thermal diffusivity D. C(r) is an instrument constant that depends on the measurement system. The C(r) depends on the relative distance r of the measurement point (which can be obtained from an image of the infrared thermographic camera 40) from the laser beam in the x direction.
An in-plane thermal conductivity k is obtained from the linear slope of laser power dependence of the maximum temperature (see reference numeral 2215 in the figure), which is obtained by measuring the maximum temperature (see reference numeral 2203) at each pixel.
Then, a specific heat capacity pCp is calculated from the above in-plane thermal diffusivity D and in-plane thermal conductivity k based on the above equation (1) (see reference numeral 2217).

<Variation 1>

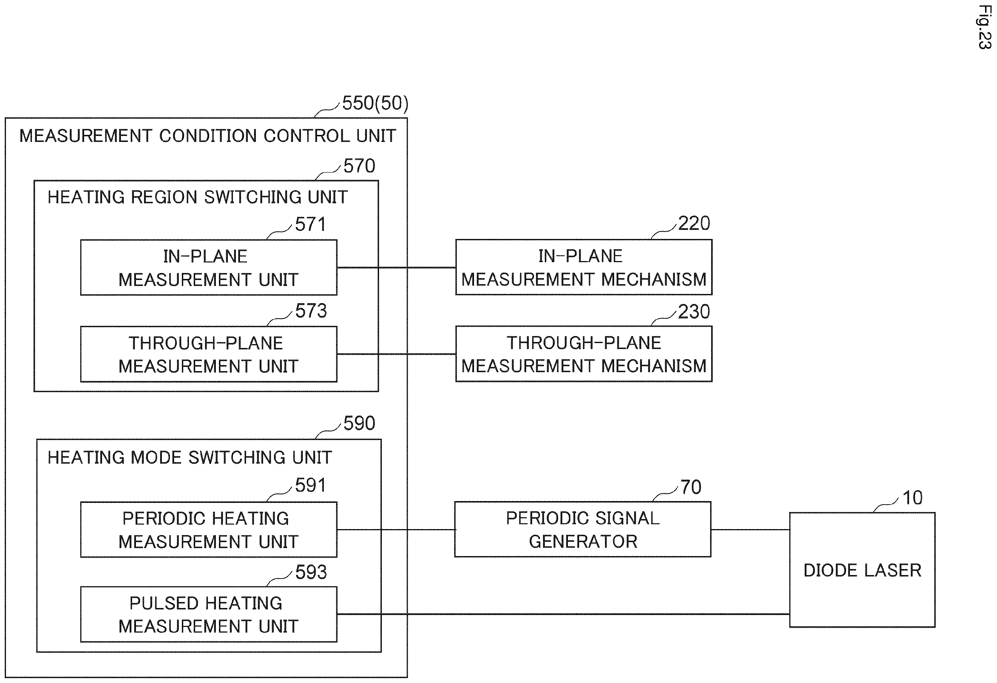

FIG. 23 illustrates a measurement condition control unit 550 according to variation 1.
The above explanation discussed performing the periodic area heating (see FIG. 1), periodic line heating (see FIG. 12), pulsed area heating (see FIG. 17), or pulsed line heating (see FIG. 20) each by the independent thermophysical property evaluation device 100 or the like. However, this is not limiting. For example, the thermophysical property evaluation device 100 may be configured to switch heating conditions, i.e., measurement conditions.
For example, as shown in FIG. 23, the computer 50 may include a measurement condition control unit 550. The measurement condition control unit 550 includes a heating region switching unit 570 to switch heating regions between that for area heating and that for line heating, and a heating mode switching unit 590 to switch heating modes between periodic heating and pulsed heating.
The heating region switching unit 570 includes an in-plane measurement unit 571 and a through-plane measurement unit 573. The in-plane measurement unit 571 controls an in-plane measurement mechanism 220. The in-plane measurement mechanism 220 performs line heating via, e.g., the light guide 210 (see FIG. 12). The through-plane measurement unit 573 controls a through-plane measurement mechanism 230. The through-plane measurement mechanism 230 performs area heating via, e.g., the light guide 20 (see FIG. 1).
The heating mode switching unit 590 includes a periodic heating measurement unit 591 and a pulsed heating measurement unit 593. The periodic heating measurement unit 591 controls the diode laser 10 via the periodic signal generator 70 to perform periodic heating. The pulsed heating measurement unit 593 controls the diode laser 10 to perform pulsed heating.

In response to receiving a designation of measurement conditions from the computer 50, the measurement condition control unit 550 switches the measurement conditions through the heating region switching unit 570 and the heating mode switching unit 590.

Switching the measurement conditions in the above manner enables a wider variety of measurements of thermophysical properties of the measurement sample 1. For example, performing both through-plane and in-plane measurements on the measurement sample 1 enables anisotropic measurement of the thermal conductivity.

<Variation 2>

In the above description, the first surface 103 (front side) of the measurement sample 1 is heated by the diode laser 10 and the second surface 105 (back side) of the measurement sample 1 is measured by the infrared thermographic camera 40. However, this is not limiting. For example, the heating of the measurement sample 1 by the diode laser 10 and the measurement of the measurement sample 1 by the infrared thermographic camera 40 may be performed on the same side of the measurement sample 1.

In the above description in relation to the examples in FIG. 1 and other figures, the diode laser 10 and the light guide 20 are used to heat the measurement sample 1. However, this is not limiting, and any other method or arrangement may be used that ensure a uniform intensity distribution on the irradiation plane of the measurement sample 1. For example, an array of multiple light emitting diodes (LEDs) may be used as the light source. Other heating methods that can heat the entire measurement sample 1, such as induction heating or resistance heating, may be used. Further, instead of at least one of the fiber 21 and the condenser 23, or in combination with the fiber 21 and the condenser 23, a diffuser plate, such as so-called frosted glass, may be used to diffuse light from the light source. In addition, a diode laser with a relatively large Gaussian distribution may be used as the diode laser 10. To further illustrate, a constant light intensity portion of the laser beam with a relatively large Gaussian distribution may be used to homogenize the intensity distribution on the irradiation plane of the measurement sample 1.

In the above description, the diaphragm 33 is provided between the measurement sample 1 and the infrared thermographic camera 40. However, an implementation without the diaphragm 33 is also possible.

In the example shown in FIG. 12 above, the laser beam emitted from the diode laser 10 to the cylindrical lens 215 is perpendicular to the first surface 103 of the measurement sample 1. However, this is not limiting. That is, the laser beam from the diode laser 10 may be emitted obliquely, provided that the heating region Hp is elongate in one direction on the first surface 103 of the measurement sample 1.

In the above embodiment, the cylindrical lens 215 is used to make the heating region Hp elongate in one direction. However, this is not limiting. For example, instead of the cylindrical lens 215, a masking member may be provided to block a portion of the laser beam to make the heating region Hp elongate in one direction. Alternatively, a sheet laser that emits sheet light may be used as a light source instead of the diode laser 10. Still alternatively, without the use of the cylindrical lens 215 or other arrangements, the laser beam may be emitted obliquely with respect to the top surface 11 of the measurement sample 1 to make the heating region Hp elongate in one direction.

The means for heating the measurement sample 1 by the diode laser 10 is not limited to laser beams. Other heating methods that can locally heat the measurement sample 1, such as induction heating or resistance heating, may also be used.

The measurement sample 1 is not limited. For example, glass, synthetic diamonds, semiconductors, polymer films, liquid crystal, etc. may be used as the measurement sample 1. When glass is used as the measurement sample 1, the inventive techniques discussed herein can detect voids or cracks formed inside the measurement sample 1. In other words, the inventive techniques can obtain information about the density of the measurement sample 1 as an evaluation of the measurement sample 1. The information about the density refers to information that can be used to determine the density of the measurement sample 1. The information about the density includes, in addition to the value of the density of the measurement sample 1, a relative evaluation of the density (e.g., coarse/dense) and the presence/absence of voids inside the measurement sample 1.

In the above embodiments, carbon fiber-reinforced resin has been used as the measurement sample 1. The type of this carbon fiber-reinforced resin is not limited and may be, for example, a recycled carbon fiber reinforced resin. For example, the measurement sample 1 may be an uncured carbon fiber-reinforced resin prior to molding (curing) by heating/pressurizing, i.e., an intermediate base material. The measurement sample 1 may be composed of multiple types of materials with mutually different thermal conductivities. Still alternatively, the measurement sample 1 may be any material other than composite materials. For example, the measurement sample 1 may be a glass, semiconductor, polymer film, liquid crystal, or the like.

Products in actual use (such as automobiles, aircrafts, or sporting goods) may also be used as the measurement sample 1. For example, for structural components of an aircraft, which employ many composite materials, fatigue tests and thermal diffusivity evaluations are conducted during a manufacturing phase on portions of the components where fatigue damages have a significant impact on airworthiness. Then, as part of maintenance tasks during heavy maintenance of the aircraft in operation, the above measurement of specific heat and other metrics can be performed on the aircraft to diagnose the fatigue condition of the portions. This is possible because the present embodiments enable a portable, non-contact, and non-destructive inspection method using thermography and laser heating. This ability to evaluate and diagnose the fatigue condition leads to the ability to determine the need to extend or shorten the design life defined by flight cycles, extending the service life and improving the reliability of the aircraft. In addition, incorporating these data into the design makes it possible to accurately estimate the design life by taking into account the effects of actual operations, contributing to extended service life. To further illustrate, for example, the above measurement of specific heat and other metrics can be performed on relevant portions of building structures (e.g., suspension ropes of bridge piers) to detect the condition of the portions. Alternatively, the above measurement of specific heat and other metrics can be performed on relevant portions of machinery (e.g., turbines), structures such as yachts, rockets, etc., or residential structures such as window sashes (fittings) to detect the condition of the portions.

In the above description, differences in thermal conductivity and specific heat from those in the reference undamaged condition is used for the fatigue evaluation. However, this is not limiting. For example, a ratio between specific heat rates or an absolute value of specific heat may be used for the fatigue evaluation. Further, instead of or in addition to using the undamaged condition as the reference, fatigue evaluation may be performed based on the condition where the fatigue life has been reached or on prior evaluation data or theoretical values. The information about the fatigue includes, in addition to values calculated as fatigue evaluations of the measurement sample 1, relative evaluation of fatigue (e.g., how far fatigue has progressed), reaching or not reaching the fatigue life, the estimated duration and number of times the sample can be used before reaching the fatigue life, the presence or absence of microcracks or micro delamination inside the measurement sample 1, etc.

The information about the thermal diffusivity, about the thermal conductivity, and about the specific heat, which is obtained in the process of calculating the information about the fatigue of the measurement sample 1, may be output and/or stored together with or instead of the information about the fatigue. The information about the thermal diffusivity, about the thermal conductivity, and about the specific heat includes, but is not limited to, the thermal conductivity distribution, values of the thermal conductivity and other metrics (including average, maximum, and minimum values), relative evaluations of the thermal conductivity and other metrics (e.g., how large or small the thermal diffusivity is), and results of comparison with prior evaluation data or theoretical values.

Further, information about the life of the measurement sample 1 may be output and/or stored together with or instead of the information about the fatigue of the measurement sample 1. The information about the life includes reaching or not reaching the fatigue life, the estimated duration and number of times the sample can be used before reaching the fatigue life, and the degree (e.g., percentage) of fatigue progress with respect to the fatigue life, etc.

The above description discussed obtaining the information about the fatigue of the measurement sample 1 associated with the tensile test by the tensile tester (not shown). However, this is not limiting. The information about the fatigue of the measurement sample 1 may be obtained from any other load test that applies loads to the measurement sample 1, such as plane bending fatigue tests, rotary bending fatigue tests, and ultrasonic fatigue tests. The tensile tester (not shown) may or may not be included in the thermophysical property evaluation device 100.

In the above description, the calculation result display unit 59 displays (outputs), on the display (not shown), the calculation results of the thermal conductivity, specific heat, and other metrics as evaluations of the sample. However, this is not limiting. For example, the calculation results of at least one of the thermal diffusivity, thermal conductivity, and specific heat may be transmitted to other devices than the computer 50 or may be stored in the computer 50.

It is not essential to output all of the information about the thermal diffusivity, about the thermal conductivity, and about the specific heat. In other words, any one of or some combination of the information about the thermal diffusivity, about the thermal conductivity, and about the specific heat may be output.

In an alternative implementation, a program of the above measurement method may be stored in a well-known thermophysical property measurement device (not shown). For example, the above measurement method may be provided over a network (not shown).

While various embodiments and variations have been described above, it will be readily understood that these embodiments and variations may be combined.

The present disclosure is not limited in any way to the above embodiments, and may be implemented in various forms without departing from the gist of the present disclosure.

The measurement sample 1 is an example of the object. The diode laser 10 is an example of the irradiation unit. The temperature amplitude distribution calculation unit 54 is an example of the output power switching unit, the first temperature distribution acquisition unit, and the second temperature distribution acquisition unit. The thermal conductivity calculation unit 56 is an example of the identification unit. The thermal diffusivity distribution calculation unit 55 is an example of the thermal diffusivity identification unit. The specific heat calculation unit 57 is an example of the specific heat identification unit and the identification unit. The fatigue evaluation calculation unit 58 is an example of the fatigue information identification unit and the identification unit.

Reference Signs List

1 Thermophysical property evaluation device
10 Diode laser
20 Light guide
21 Fiber
23 Condenser
39 Diaphragm
40 Infrared thermographic camera
50 Computer
55 Thermal diffusivity distribution calculation unit
56 Thermal conductivity calculation unit
57 Specific heat calculation unit

**Claims**

1. A device comprising:

   an irradiation unit configured to periodically irradiate an object with light to heat the object;
   an output power switching unit configured to switch an output power of the light with which the object is irradiated by the irradiation unit at least between a first output power and a second output power, the first output power providing a first maximum output power during one period, the second output power providing a second maximum output power during one period;
   a first temperature distribution acquisition unit configured to obtain a first temperature distribution of the object, the first temperature distribution being a temperature distribution of the object heated by the light of the first output power;
   a second temperature distribution acquisition unit configured to obtain a second temperature distribution of the object, the second temperature distribution being a temperature distribution of the object heated by the light of the second output power; and
   an identification unit configured to identify information about thermal conductivity of the object based on the first temperature distribution and the second temperature distribution.

2. The device according to claim 1, wherein the identification unit is configured to:

   obtain a first temperature amplitude distribution of the object based on the first temperature distribution, the first temperature amplitude distribution being a temperature amplitude distribution of the object heated by the light of the first output power;
   obtain a second temperature amplitude distribution of the object based on the second temperature distribution, the second temperature amplitude distribution being a temperature amplitude distribution of the object heated by the light of the second output power; and
   identify the information about thermal conductivity of the object based on the first temperature amplitude distribution and the second temperature amplitude distribution.

3. The device according to claim 1 or 2, wherein the identification unit is configured to:

   obtain a first maximum temperature distribution of the object based on the first temperature distribution, the first maximum temperature distribution being a distribution of maximum temperatures at respective locations on the object heated by the light of the first output power;
   obtain a second maximum temperature distribution of the object based on the second temperature distribution, the second maximum temperature distribution being a distribution of maximum temperatures at respective locations on the object heated by the light of the second output power; and
   identify the information about thermal conductivity of the object based on the first maximum temperature distribution and the second maximum temperature distribution.

4. The device according to any one of claims 1 to 3, further comprising a thermal diffusivity identification unit configured to identify information about thermal diffusivity of the object based on responses of changes over time in the temperature distribution of the object heated by the light of the first output power.

5. The device according to any one of claims 1 to 4, further comprising a specific heat identification unit configured to identify information about specific heat of the object based on the information about thermal conductivity of the object identified by the identification unit.

6. The device according to any one of claims 1 to 5, further comprising a fatigue information identification unit configured to identify information about fatigue of the object based on the information about thermal conductivity of the object identified by the identification unit.

7. The device according to any one of claims 1 to 6, wherein

the irradiation unit is configured to irradiate the object over a wider range than a region of the object in which the temperature distribution is measured, and
the identification unit is configured to identify information about through-plane thermal conductivity of the object.

8. The device according to any one of claims 1 to 7, wherein

the irradiation unit is configured to irradiate a region of the object extending in one direction on a surface of the object, and
the identification unit is configured to identify information about in-plane thermal conductivity of the object.

9. The device according to any one of claims 1 to 8, wherein

the irradiation unit is configured to switch between a first mode and a second mode, the first mode being a mode in which the irradiation unit irradiates the object over a wider range than a region of the object in which the temperature distribution is measured, the second mode being a mode in which the irradiation unit irradiates a region of the object extending in one direction on a surface of the object, and
the identification unit is configured to identify information about through-plane thermal conductivity of the object in the first mode and to identify information about in-plane thermal conductivity of the object in the second mode.

10. A method comprising:

periodically irradiating an object with light to heat the object;
switching an output power of the light with which the object is irradiated at least between a first output power and a second output power, the first output power providing a first maximum output power during one period, the second output power providing a second maximum output power during one period;
obtaining a first temperature distribution of the object, the first temperature distribution being a temperature distribution of the object heated by the light of the first output power;
obtaining a second temperature distribution of the object, the second temperature distribution being a temperature distribution of the object heated by the light of the second output power; and
identifying information about thermal conductivity of the object based on the first temperature distribution and the second temperature distribution.

11. A program for causing a computer to execute functions of:

periodically irradiating an object with light to heat the object;
switching an output power of the light with which the object is irradiated at least between a first output power and a second output power, the first output power providing a first maximum output power during one period, the second output power providing a second maximum output power during one period;
obtaining a first temperature distribution of the object, the first temperature distribution being a temperature distribution of the object heated by the light of the first output power;
obtaining a second temperature distribution of the object, the second temperature distribution being a temperature distribution of the object heated by the light of the second output power; and
identifying information about thermal conductivity of the object based on the first temperature distribution and the second temperature distribution.

12. A device comprising:

an irradiation unit configured to periodically irradiate an object with light to heat the object;

an output power switching unit configured to switch an output power of the light with which the object is irradiated by the irradiation unit at least between a first output power and a second output power, the first output power providing a first maximum output power during one period, the second output power providing a second maximum output power during one period;

a first temperature distribution acquisition unit configured to obtain a first temperature distribution of the object, the first temperature distribution being a temperature distribution of the object heated by the light of the first output power;

a second temperature distribution acquisition unit configured to obtain a second temperature distribution of the object, the second temperature distribution being a temperature distribution of the object heated by the light of the second output power; and

an identification unit configured to identify information about specific heat of the object based on the first temperature distribution and the second temperature distribution.

13. A method comprising:

periodically irradiating an object with light to heat the object;

switching an output power of the light with which the object is irradiated at least between a first output power and a second output power, the first output power providing a first maximum output power during one period, the second output power providing a second maximum output power during one period;

obtaining a first temperature distribution of the object, the first temperature distribution being a temperature distribution of the object heated by the light of the first output power;

obtaining a second temperature distribution of the object, the second temperature distribution being a temperature distribution of the object heated by the light of the second output power; and

identifying information about specific heat of the object based on the first temperature distribution and the second temperature distribution.

14. A program for causing a computer to execute functions of:

periodically irradiating an object with light to heat the object;

switching an output power of the light with which the object is irradiated at least between a first output power and a second output power, the first output power providing a first maximum output power during one period, the second output power providing a second maximum output power during one period;

obtaining a first temperature distribution of the object, the first temperature distribution being a temperature distribution of the object heated by the light of the first output power;

obtaining a second temperature distribution of the object, the second temperature distribution being a temperature distribution of the object heated by the light of the second output power; and

identifying information about specific heat of the object based on the first temperature distribution and the second temperature distribution.

15. A device comprising:

an irradiation unit configured to periodically irradiate an object with light to heat the object;

an output power switching unit configured to switch an output power of the light with which the object is irradiated by the irradiation unit at least between a first output power and a second output power, the first output power providing a first maximum output power during one period, the second output power providing a second maximum output power during one period;

a first temperature distribution acquisition unit configured to obtain a first temperature distribution of the object, the first temperature distribution being a temperature distribution of the object heated by the light of the first output power;

a second temperature distribution acquisition unit configured to obtain a second temperature distribution of the object, the second temperature distribution being a temperature distribution of the object heated by the light of the second output power; and

an identification unit configured to, based on the first temperature distribution and the second temperature distribution, calculate thermal conductivity of the object to identify information about fatigue of the object.

16. A method comprising:

periodically irradiating an object with light to heat the object;

switching an output power of the light with which the object is irradiated at least between a first output power and a second output power, the first output power providing a first maximum output power during one period, the second output power providing a second maximum output power during one period;

obtaining a first temperature distribution of the object, the first temperature distribution being a temperature distribution of the object heated by the light of the first output power;

obtaining a second temperature distribution of the object, the second temperature distribution being a temperature distribution of the object heated by the light of the second output power; and

based on the first temperature distribution and the second temperature distribution, calculating thermal conductivity of the object to identify information about fatigue of the object.

17. A program for causing a computer to execute functions of:

periodically irradiating an object with light to heat the object;

switching an output power of the light with which the object is irradiated at least between a first output power and a second output power, the first output power providing a first maximum output power during one period, the second output power providing a second maximum output power during one period;

obtaining a first temperature distribution of the object, the first temperature distribution being a temperature distribution of the object heated by the light of the first output power;

obtaining a second temperature distribution of the object, the second temperature distribution being a temperature distribution of the object heated by the light of the second output power; and

based on the first temperature distribution and the second temperature distribution, calculating thermal conductivity of the object to identify information about fatigue of the object.

Fig.1

(A)

50

TEMPERATURE DISTRIBUTION DATA
PERIODIC SIGNALS

100

PERIODIC SIGNALS

70

40

PERIODIC SIGNALS

z

x

10

105

33

30

31

103

1

21

25

23

20

(B)

LIGHT INTENSITY
(a.u.)

252

191

131

83

10

Fig.2

Fig.3

50

```
┌─────────────────────────────────────────────────────────┐
│                                                           │
│      501                          505                     │
│   ┌──────────────┐          ┌──────────────┐              │
│   │     CPU      │──────────│     HDD      │              │
│   └──────────────┘          └──────────────┘              │
│                                                           │
│      503                          507                     │
│   ┌──────────────┐          ┌────────────────────┐        │
│   │ MAIN MEMORY  │──────────│ COMMUNICATION I/F   │        │
│   └──────────────┘          └────────────────────┘        │
│                                                           │
└─────────────────────────────────────────────────────────┘
```

Fig.4

(A) f dependence

(A-1) Input signal

(A-2)

(A-3)

(A-4) $\dfrac{\partial A}{\partial f} \propto \dfrac{C}{D}$

(A-5) $\dfrac{\partial \varphi}{\partial f} \propto \dfrac{C}{D}$

(B) P dependence

(B-1) Input signal

(B-2)

(B-3)

(B-4) $\dfrac{\partial A}{\partial P} \propto \dfrac{C}{k}$

(B-5)

Fig.5

(A)

(B)

Fig.6

Fig.7

START

TENSILE TEST — S701

FATIGUE EVALUATION — S702

S703

TERMINATE TENSILE TEST? — NO

YES

OUTPUT CALCULATION RESULTS — S704

END

Fig.8

```
┌──────────────────────────────────────────────────┐
│              FATIGUE EVALUATION                    │
└──────────────────────────────────────────────────┘
                        │
                        ▼
┌──────────────────────────────────────────────────┐
│        CALCULATE PHASE LAG DISTRIBUTION            │──── S801
└──────────────────────────────────────────────────┘
                        │
                        ▼
┌──────────────────────────────────────────────────┐
│     CALCULATE THERMAL DIFFUSIVITY DISTRIBUTION     │──── S802
└──────────────────────────────────────────────────┘
                        │
                        ▼
┌──────────────────────────────────────────────────┐
│                 SET FREQUENCY                      │──── S803
└──────────────────────────────────────────────────┘
                        │
                        ▼
┌──────────────────────────────────────────────────┐
│   CALCULATE TEMPERATURE AMPLITUDE DISTRIBUTION     │──── S804
└──────────────────────────────────────────────────┘
                        │
                        ▼
┌──────────────────────────────────────────────────┐
│    CALCULATE THERMAL CONDUCTIVITY DISTRIBUTION     │──── S805
└──────────────────────────────────────────────────┘
                        │
                        ▼
┌──────────────────────────────────────────────────┐
│        CALCULATE SPECIFIC HEAT DISTRIBUTION        │──── S806
└──────────────────────────────────────────────────┘
                        │
                        ▼
┌──────────────────────────────────────────────────┐
│          CALCULATE FATIGUE EVALUATION              │──── S807
└──────────────────────────────────────────────────┘
                        │
                        ▼
                  (    END    )
```

Fig.9

Fig.10

Fig.11

Fig.12

(A)

TEMPERATURE DISTRIBUTION DATA
PERIODIC SIGNALS

50

200

PERIODIC SIGNALS

70

40

PERIODIC SIGNALS

110

105

310

103

215

210

213

211

211

Z

X

(B)

1

HP

105

103

215

Z

Y

X

Fig.13

Fig.14

(A)

(A-1)

HP

105

103

1

L2

z

y

x

(A-2)

Top view

HP

y

x

(B)

Side view

d

d

0

z

x

x

Fig.15

EP 4 471 414 A1

$$D\varphi = \frac{\pi f}{\left(\frac{\partial \varphi}{\partial x}\right)^2}$$

$$D = \sqrt{D_A D\varphi}$$

$$A = Const.e^{-\sqrt{\frac{\pi f}{D_A}}\, x}$$

$$k = \frac{2}{\pi \frac{\partial A}{\partial P}} \sqrt{\frac{D}{2\pi f}}$$

$$\rho Cp = \frac{k}{D}$$

Fig.16

Fig.17

TEMPERATURE DISTRIBUTION DATA

TRIGGER SIGNAL

50

300

40

105  33  30

31

103

1

10

Z

X

21

25

23

20

Fig.18

EP 4 471 414 A1

(A) input signal   (B) t dependence   (C) P dependence   (D) P dependence

Fig.19

Fig.20

(A)

TEMPERATURE DISTRIBUTION DATA

TRIGGER SIGNAL

Fig.21

(A) input signal

(B) t dependence

(C) P dependence
at the heating line

(D) P dependence
at the heating line

$\dfrac{\partial T}{\partial t} \propto \dfrac{C}{D}$

$P=\text{const}$

$\dfrac{\partial T_{max}}{\partial p} \propto \dfrac{C}{k}$

Fig.22

Fig.23

EP 4 471 414 A1

550(50) MEASUREMENT CONDITION CONTROL UNIT

570 HEATING REGION SWITCHING UNIT

571 IN-PLANE MEASUREMENT UNIT

573 THROUGH-PLANE MEASUREMENT UNIT

220 IN-PLANE MEASUREMENT MECHANISM

230 THROUGH-PLANE MEASUREMENT MECHANISM

590 HEATING MODE SWITCHING UNIT

591 PERIODIC HEATING MEASUREMENT UNIT

593 PULSED HEATING MEASUREMENT UNIT

70 PERIODIC SIGNAL GENERATOR

10 DIODE LASER

# EP 4 471 414 A1

**INTERNATIONAL SEARCH REPORT**

International application No.

**PCT/JP2023/002541**

| | |
|---|---|
| **A.** | **CLASSIFICATION OF SUBJECT MATTER** |

**G01N 25/18**(2006.01)i
FI:   G01N25/18 H

According to International Patent Classification (IPC) or to both national classification and IPC

| | |
|---|---|
| **B.** | **FIELDS SEARCHED** |

Minimum documentation searched (classification system followed by classification symbols)

   G01N25/18

Documentation searched other than minimum documentation to the extent that such documents are included in the fields searched

   Published examined utility model applications of Japan 1922-1996
   Published unexamined utility model applications of Japan 1971-2023
   Registered utility model specifications of Japan 1996-2023
   Published registered utility model applications of Japan 1994-2023

Electronic data base consulted during the international search (name of data base and, where practicable, search terms used)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | JP 2015-68789 A (NICHIAS CORP) 13 April 2015 (2015-04-13)<br>entire text, all drawings | 1-17 |
| A | JP 2007-225559 A (KOBE STEEL LTD) 06 September 2007 (2007-09-06)<br>entire text, all drawings | 1-17 |
| A | JP 2013-3069 A (MITSUBISHI CHEMICALS CORP) 07 January 2013 (2013-01-07)<br>entire text, all drawings | 1-17 |
| A | JP 2011-185852 A (NATIONAL INSTITUTE OF ADVANCED INDUSTRIAL SCIENCE& TECHNOLOGY) 22 September 2011 (2011-09-22)<br>entire text, all drawings | 1-17 |
| A | JP 3-189547 A (HITACHI LTD) 19 August 1991 (1991-08-19)<br>entire text, all drawings | 1-17 |
| A | JP 2006-329969 A (NATIONAL INSTITUTE OF ADVANCED INDUSTRIAL SCIENCE& TECHNOLOGY) 07 December 2006 (2006-12-07)<br>entire text, all drawings | 1-17 |

☑ Further documents are listed in the continuation of Box C.        ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **03 April 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
|---|
| **PCT/JP2023/002541** |

**C.** **DOCUMENTS CONSIDERED TO BE RELEVANT**

| Category* | Citation of document, with indication, where appropriate, of the relevant passages | Relevant to claim No. |
|---|---|---|
| A | JP 2022-500624 A (UNIVERSITY OF VIRGINIA PATENT FOUNDATION) 04 January 2022 (2022-01-04)<br>entire text, all drawings | 1-17 |
| A | JP 2018-25560 A (UNIV NAGOYA) 15 February 2018 (2018-02-15)<br>entire text, all drawings | 1-17 |
| A | CN 104155336 A (TSINGHUA UNIVERSITY) 19 November 2014 (2014-11-19)<br>entire text, all drawings | 1-17 |

Form PCT/ISA/210 (second sheet) (January 2015)

## INTERNATIONAL SEARCH REPORT
### Information on patent family members

International application No.

**PCT/JP2023/002541**

| Patent document cited in search report | | | Publication date (day/month/year) | Patent family member(s) | Publication date (day/month/year) |
|---|---|---|---|---|---|
| JP | 2015-68789 | A | 13 April 2015 | (Family: none) | |
| JP | 2007-225559 | A | 06 September 2007 | (Family: none) | |
| JP | 2013-3069 | A | 07 January 2013 | (Family: none) | |
| JP | 2011-185852 | A | 22 September 2011 | (Family: none) | |
| JP | 3-189547 | A | 19 August 1991 | (Family: none) | |
| JP | 2006-329969 | A | 07 December 2006 | (Family: none) | |
| JP | 2022-500624 | A | 04 January 2022 | US 022/0146443 A1 entire text, all drawings EP 3827245 A1 KR 10-2021-0048539 A | |
| JP | 2018-25560 | A | 15 February 2018 | (Family: none) | |
| CN | 104155336 | A | 19 November 2014 | (Family: none) | |

Form PCT/ISA/210 (patent family annex) (January 2015)

**EP 4 471 414 A1**

**Patent documents cited in the description**

- JP 2002090322 A